# EUROPEAN PATENT APPLICATION

(11) **EP 3 399 015 A1**
(43) Date of publication of application: **07.11.2018**
(21) Application number: 16881829.2
(22) Date of filing: 28.12.2016
(51) Int. Cl.: C12M 1/08, C12P 5/00

(54) **GAS STIRRING FERMENTATION DEVICE**

(30) Priority: 28.12.2015 JP 2015256881
(71) Applicant: Ajinomoto Co., Inc., Tokyo 104-8315 (JP)
(72) Inventor: MATSUKA, Shinichiro, Saga-shi Saga 840-2105 (JP); NITTA, Nobuhisa, Kawasaki-shi Kanagawa 210-8681 (JP)
(74) Representative: Grünecker Patent- und Rechtsanwälte PartG mbB
(86) International application number: PCT/JP2016/089141
(87) International publication number: WO 2017/115855

(57) **Abstract**

Provided is a gas stirring fermentation device having excellent mixing performance of a culture liquid and being applicable to aerobic culture requiring a high demanded oxygen amount. The gas stirring fermentation device includes a culture tank, a draft tube located inside the culture tank, and a gas supply pipe supplying gas to the inside of the culture tank, in which the gas supply pipe is provided with a gas discharge part made of a sintered metal film, and a culture liquid in the culture tank can be stirred by the gas discharged from the gas discharge part.

## Description

### Field

The present invention relates to a gas stirring fermentation device. Further, the present invention relates to a method for producing chemical substances.

### Background

A fermentation method is known as one of the methods for producing chemical substances. For example, Patent Literature 1 has disclosed a method for producing isoprene by culturing microorganisms having isoprene-producing ability represented by hay bacillus (*Bacillus subtilis*).

Among chemical substances that can be produced by the fermentation method, flammable substances such as isoprene described above are also included. When the flammable substance is a production target, the specification of a fermentation device that takes the danger of fire and explosion due to continuation of combustion into consideration is required. As factors causing combustion and explosion, three elements of flammable substances, oxygen, and an ignition source exist and, in order to prevent combustion and explosion, at least one of these three elements is required to be eliminated. However, when the flammable substance such as isoprene is produced by the fermentation method, possibility of combustion and explosion (hereinafter also may be referred to as "explosion and the like") is concerned because the three elements of "the flammable substance being the production target", "oxygen", and "electric energy of a stirrer to be an ignition source" exist.

As a fermentation device that reduces the possibility of the explosion and the like, a fermentation device not using a conventional mechanical stirrer can be considered. For example, Non-Patent Literature 1 has disclosed an air-lift type reactor that can discharges gas from a nozzle provided in the lower part of a culture tank and the gas stirs the culture liquid in the culture tank.

In addition, Patent Literature 2 has disclosed a technique of dispersing and supplying air, oxygen or, mixed gas thereof to a culture tank using a diffusing tube having a sintered metal film to carry out aerobic culture.

### Citation List

### Patent Literature

Patent Literature 1: U. S. Patent No. 5849970
Patent Literature 2: Japanese Patent No. 4200655 Non Patent Literature

Non Patent Literature 1: Yoshiyuki Bando "Draft tube tuki kihoutouno ryuudoutokuseito sono seinoukaizen (Flow Characteristics in Bubble Column with Draft Tube and Performance Improvement)," Kagaku Kogaku Ronbunshu (Chemical Engineering Papers) Vol. 27, No. 4, p. 430-441 Summary

### Technical Problem

In the technique described in Non-Patent Literature 1, the gas discharged from the nozzle forms air bubbles in the culture tank. By the movement of such bubbles, the culture liquid in the culture tank is stirred. As a result of attempted application of such a technique in the production of chemical substances by the fermentation method, the inventors of the present invention have found that the chemical substances cannot be sufficiently produced in some cases due to deterioration in mixing performance of a culture liquid as compared with the mixing performance of a conventional fermentation device using a mechanical stirrer. Moreover, when the aerobic culture is carried out using the technique described in Non-Patent Literature 1, oxygen-containing gas is required to be discharged from the nozzle, the culture liquid is required to be stirred, and oxygen is required to be supplied to the culture liquid. However, the inventors of the present invention also have found that the shearing and dispersing action of bubbles is lowered and the oxygen supply performance to the culture liquid may be remarkably deteriorated as compared with the oxygen supply performance of the conventional fermentation device in which the culture liquid is stirred by a mechanical stirrer.

The technique described in Patent Literature 2 does not exclude the use of a mechanical stirrer, and thus there is concern about the possibility of explosion and the like when the flammable substance is produced.

An object of the present invention is to provide a gas stirring fermentation device having excellent mixing performance of a culture liquid, in particular, to provide a gas stirring fermentation device that can be applied to aerobic culture with the high demanded oxygen amount. Solution to Problem

As a result of intensive studies on the above problems, the inventors of the present invention have found that the above problems can be solved by a gas stirring fermentation device having the following specific constitution and have completed the present invention.

That is, the present invention includes the following embodiments.
[1] A gas stirring fermentation device comprising:
   a culture tank;
   a draft tube located inside the culture tank; and
   a gas supply pipe supplying gas to inside of the culture tank, wherein
   the gas supply pipe is provided with a gas discharge part made of a sintered metal film, and
   a culture liquid in the culture tank is capable of being stirred by gas discharged from the gas discharge part.
[2] The device according to [1], wherein the sintered metal film has an average pore diameter of 20 µm or less.
[3] The device according to [1] or [2], wherein a discharge gas linear velocity (Gas discharge amount [m³/s]/Surface area [m²] of sintered metal film) at the gas discharge part made of the sintered metal film is 0.04 m/s or less.
[4] The device according to any of [1] to [3], wherein D₁ and D₂ satisfy the relation of 0.7<D₂/D₁ where D₁ is the inner diameter of the culture tank and D₂ is the inner diameter of the draft tube.
[5] A method for producing a chemical substance, the method comprising:
   a step of discharging gas from a gas discharge part made of a sintered metal film into a culture liquid including microorganisms having ability to produce the chemical substance and passing the culture liquid including the discharged gas through inside of a draft tube to stir the culture liquid, and
   a step of culturing the microorganisms to produce the chemical substance.
[6] The method according to [5], wherein the chemical substance comprises a flammable substance.
[7] The method according to any of [5] or [6], wherein the chemical substance is a hydrophobic substance.
[8]. The method according to any of [5] to [7], wherein the chemical substance is an isoprenoid compound.
[9] The method according to any of [5] to [8], wherein the sintered metal film has an average pore diameter of 20 µm or less.
[10] The method according to any of [5] to [9], wherein a discharge gas linear velocity (Gas discharge amount [m³/s]/Surface area [m²] of sintered metal film) at the gas discharge part made of the sintered metal film is 0.04 m/s or less.

### Advantageous Effects of Invention

According to the present invention, a gas stirring fermentation device having excellent mixing performance of a culture liquid, in particular, a gas stirring fermentation device that can applied to aerobic culture with the high demanded oxygen amount.

### Brief Description of Drawings

FIG. 1 is a schematic view illustrating a gas stirring fermentation device according to an embodiment of the present invention.
FIG. 2 is a schematic view for illustrating a circulation flow of a culture liquid in the gas stirring fermentation device according to an embodiment of the present invention.
FIG. 3 is a schematic view for illustrating suitable sizes and arrangements of the gas stirring fermentation device of the present invention.
FIG. 4 is a chart illustrating a pAH162-Para-mvaES plasmid having an E. faecalis-derived mvaES operon under the control of an *E. coli Para* promoter and a repressor gene araC.
FIG. 5 is a chart illustrating a map of pAH162-mvaES.
FIG. 6 is a chart illustrating a plasmid for pAH162-MCS-mvaES chromosome immobilization.
FIG. 7 is a chart illustrating a set of plasmids for chromosome immobilization holding an mvaES gene under transcriptional control of (A) P_{11dD}, (B) P_{phoC}, or (C) P_{pstS}.
FIG. 8 is a chart illustrating the outline of formulation of a pAH162-λattL-KmR-λattR vector.
FIG. 9 is a chart illustrating an expression vector for pAH162-Ptac chromosome immobilization.
FIG. 10 is a chart illustrating codon optimization in a KDyI operon obtained by chemical synthesis.
FIG. 11 is a chart illustrating chromosome-immobilized plasmids of (A) pAH162-Tc-Ptac-KDyI and (B) pAH162-Km-Ptac-KDyI that hold the codon-optimized KDyI operon.
FIG. 12 is a chart illustrating a chromosome-immobilized plasmid holding a mevalonate kinase gene derived from *M*. *paludicola.*
FIG. 13 is a chart illustrating a map of genomic variants of (A) ΔampC::attBₚₕᵢ₈₀, (B) ΔampH::attBₚₕᵢ₈₀, and (C) Δcrt::attBₚₕᵢ₈₀.
FIG. 14 is a chart illustrating a map of genome modification products of A) Δcrt::pAH162-P_{tac}-mvk (X) and (B) Δcrt::P_{tac}-mvk (X).
FIG. 15 is a chart illustrating a map of chromosome modification products of (A) ΔampH::pAH162-Km-P_{tac}-KDyI, (B) ΔampC::pAH162-Km-P_{tac}-KDyI, and (C) ΔampC::P_{tac}-KDyI
FIG. 16 is a chart illustrating a map of chromosome modification products of (A) ΔampH::pAH162-Px-mvaES and (B) ΔampC::pAH162-Px-mvaES.
FIG. 17 is a chart illustrating (A) growth and a (B) isoprene production amount (mg/batch) by culturing an isoprene-producing microorganism SWITCH-PphoCAgcd/IspSM strain.
FIG. 18 is a chart illustrating a dissolved oxygen (DO) concentration in the culture of the isoprene-producing microorganism SWITCH-PphoCAgcd/IspSM strain.

### Description of Embodiments

Hereinafter, the present invention will be described in detail with reference to suitable embodiments thereof.

### [Gas stirring fermentation device]

The gas stirring fermentation device of the present invention includes:
a culture tank;
a draft tube located inside the culture tank; and
a gas supply pipe supplying gas to the inside of the culture tank,
in which the gas supply pipe is provided with a gas discharge part made of a sintered metal film, and
a culture liquid in the culture tank is capable of being stirred by the gas discharged from the gas discharge part.

Different from a conventional fermentation device equipped with a mechanical stirrer, the gas stirring fermentation device is a fermentation device in which the culture liquid is stirred only by the flow action of gas. In the gas stirring fermentation device, when gas is discharged from the gas discharge part located in the culture tank, air bubbles composed of the discharged gas are generated. Generally, the gas discharge part is located in the lower part of the culture tank and the generated bubbles rise in the culture tank. When the bubbles rise in the culture tank, the bubbles tend to gather at the center of the culture tank due to the wall effect of the culture tank. As a result, a difference in the apparent density of the culture liquid between the center part in the culture tank and the outside of the center part (wall surface side) is generated. Due to this apparent density difference, the culture liquid flows upward together with the bubbles in the center part of the culture tank and flows downward at the outer part (wall surface side) of the center part. This generates a circulation flow of the culture liquid inside the culture tank and thus the culture liquid is stirred.

Locating the draft tube inside the culture tank allows the apparent density difference to be larger and stirring of the culture liquid to be promoted. Specifically, the gas discharge part and the draft tube are aligned so that the culture liquid having a low apparent density by including more bubbles passes through inside the draft tube. This allows the difference in the apparent density of the culture liquid between the inside and the outside of the draft tube to be increased and the stirring of the culture liquid to be promoted. Here, the upward flow of the culture liquid occurs inside the draft tube (hereinafter, the action of generating such upward flow of the culture liquid is also referred to as "gas lift action") and the downward flow of the culture liquid occurs outside the draft tube.

The present invention achieves excellent mixing performance of the culture liquid, which cannot be achieved by the conventional gas stirring fermentation device, by using a gas discharge part made of a sintered metal film as the gas discharge part in a gas stirring fermentation device in which the draft tube is located inside the culture tank. The present invention further achieves excellent oxygen supply performance in such an extent that the oxygen supply performance is applicable to the aerobic culture with the high demanded oxygen amount when oxygen-containing gas is used as the gas discharged from the gas discharge part.

The gas stirring fermentation device of the present invention is suitable for producing various chemical substances, particularly hydrophobic substances. The hydrophobic substances have low solubility in the culture liquid and can be easily separated and recovered. For example, when the specific gravity of the hydrophobic substance is higher than that of the culture liquid, the hydrophobic substance may be recovered from the lower part of the culture tank. When the specific gravity of the hydrophobic substance is lower than that of the culture liquid, the hydrophobic substance may be recovered from the upper part of the culture tank. In the present invention, the "hydrophobic substance" refers to a substance exhibiting low solubility to a culture liquid to such an extent that the substance can be easily separated from the culture liquid to be used. The solubility (at normal temperature) of the hydrophobic substance in the culture liquid is preferably 10 g/kg or less, more preferably 5 g/kg or less, 1 g/kg or less, or 0.1 g/kg or less because the separation and the recover are easy.

The hydrophobic substance being the production target is not particularly limited as long as the substance can be produced by a fermentation method and examples thereof include hydrogen; saturated hydrocarbons such as methane; unsaturated hydrocarbons such as ethylene, propylene, butadiene, isobutene, and isoprene; isoprenoid compounds such as 1,3-amorphadiene, farnesene, limonene, carvone, linalool, nootkatone, and valencene; aromatic compounds such as benzaldehyde, cinnamaldehyde, vanillin, eugenol, anethole, and trimethylpyrazine; lipid compounds such as ceramide; lactone compounds such as nonalactone and decalactone; organic acid ester compounds such as isopentyl acetate, menthyl lactate, monomenthyl succinate; and sugar alcohol compounds such as xylitol and arabitol.

The gas stirring fermentation device of the present invention is also suitable for producing hydrophilic or water-miscible substances. In the present invention, the "hydrophilic or water-miscible substance" refers to a material having high solubility or water-miscibility to the culture liquid to such an extent that purification treatment such as distillation treatment and membrane separation treatment is required for separating the substance from the culture liquid to be used. The solubility (at normal temperature) of the hydrophilic or water-miscible substance with respect to the culture liquid is more than 10 g/kg, usually 20 g/kg or more, or 30 g/kg or more. Such a hydrophilic or water-miscible substance is not particularly limited as long as the substance can be produced by the fermentation method and examples of the substance include lower (C₁-C₆) alcohol compounds such as ethanol and propanol.

The isoprenoid compounds can be synthesized through two different metabolic pathways that converge to IPP and its isomer DMAPP. For example, the isoprenoid compound is composed of one or more isoprene units having a molecular formula of (C₅H₈)ₙ. The precursor of the isoprene unit is isopentenyl pyrophosphate or dimethylallyl pyrophosphate. 30,000 isoprenoid compounds have been identified and new compounds have been being identified. Isoprenoids are also known as terpenoids. The difference between the terpenes and the terpenoids is in that the terpenes are hydrocarbons whereas the terpenoids contain additional functional groups. The terpenes are classified according to the number of isoprene units in a molecule [for example, hemiterpenes (C5), monoterpenes (C10), sesquiterpenes (C15), diterpenes (C20), sesterterpens (C25), triterpenes (C30) sesquarterpenes (C35), tetraterpenes (C40), polyterpenes, and norisoprenoids]. Examples of the monoterpenes include pinene, nerol, citral, camphor, menthol, limonene, carvone, and linalool. Examples of sesquiterpenes include nerolidol and farnesol. Examples of the diterpenes include phytol and Vitamin A1. Squalene is an example of the triterpenes and carotene (Provitamin A1) is a tetraterpene (Nature Chemical Biology 2, 674-681 (2006); Nature Chemical Biology 5, 283-291 (2009); Nature Reviews Microbiology 3, 937-947 (2005); Adv Biochem Eng Biotechmol (DOI: 10.1007/10_2014_288)). Preferably, the isoprenoid compound is isoprene (monomer).

The microorganism having ability to produce isoprenoid compounds (hereinafter also referred to as "isoprenoid compound-producing microorganism") has dimethylallyl diphosphate supply pathway. Dimethylallyl diphosphate (DMAPP) is a precursor of peptidoglycan and electron acceptors (such as menaquinone) and is known to be essential for the growth of microorganisms (Fujisaki et al., J. Biochem., 1986; 99: 1137-1146). Examples of the dimethylallyl diphosphate supply pathway include a methylerythritol phosphate (MEP) pathway and a mevalonic acid (MVA) pathway.

DMAPP, a material of the isoprenoid compound (for example, a substrate for isoprene synthesis), is usually biosynthesized by either the methylerythritol phosphate pathway or the mevalonic acid pathway inherently or natively held by the microorganism. Therefore, from the viewpoint of the supply of DMAPP for efficient production of the isoprenoid compounds, the isoprenoid compound-producing microorganism used in the present invention may have enhanced methylerythritol phosphate pathway and/or mevalonic acid pathway as described later.

The isoprenoid compound-producing microorganisms are preferably aerobic microorganisms. The isoprenoid compound-producing microorganisms are preferably cultured under aerobic conditions. When the microorganisms are cultured under the aerobic conditions, the concentration of dissolved oxygen in the culture medium may be a concentration that is sufficiently higher than the growth of the microorganisms. The concentration of the dissolved oxygen in the culture medium that is sufficient for the growth of the aerobic microorganisms is not particularly limited as long as the concentration is a concentration capable of promoting the growth of aerobic microorganisms and the concentration is, for example, 1 ppm or more, 3 ppm or more, 5 ppm or more, 7 ppm or more, or 7.22 ppm or more. The concentration of the dissolved oxygen for the growth of aerobic microorganisms may also be, for example, 0.3 ppm or less, 0.15 ppm or less, or 0.05 ppm or less.

The gas stirring fermentation device of the present invention is widely applicable to the production of the chemical substances by the fermentation method. When the chemical substances to be produced include flammable substances, the specification of the fermentation device that takes the danger of fire and explosion due to continuation of combustion into consideration is required. When auxiliary agents such as organic solvents used for the production include flammable substance, the specification of the fermentation device that takes the danger of fire and explosion due to continuation of combustion into consideration is also required. The gas stirring fermentation device of the present invention is also effective in the case where the auxiliary agents such as organic solvents used for production have inflammability, ignitability, or explosibility in addition to the case where the chemical substance being the production target itself has inflammability, ignitability, or explosibility. The explosibility of the substance and auxiliary agent can be examined by a general test method for measuring the explosion limit of flammable gas and vapor. As the test method, mixed gas is put in the explosion test container and the ignition source is operated to detect the presence or absence of explosion by a temperature sensor and a pressure sensor. By changing the concentration of the mixed gas to repeat the same operation, the explosion range can be obtained (Standard Practice for Determining Limits of Flammability of Chemicals at Elevated Temperature and Pressure, ASTM E 918-83 (2011)). As a method for measuring the flash point of the substance and the auxiliary agent, for example, JIS K 2265 in JIS standard has been known. As a method for measuring the ignition point of the substance and auxiliary agent, for example, a method defined in "Standard Test Method for Autoignition Temperature of Liquid Chemicals" by ASTM International (ASTM E659-1978) has been known. As a method for measuring the boiling points of the substance and auxiliary agent, for example, a method in accordance with the "Equilibrium reflux boiling point test method" defined in 7.1 of JIS standard "Non-petroleum base motor vehicle brake fluids" (JIS K 2233-1989) has been known.

In the chemical substances, when the chemical substance being the production target includes a flammable substance and the flammable substance is produced by the aerobic culture, the possibility of explosion and the like is concerned. In particular, when the flammable substance includes flammable gas, the flammable gas and oxygen easily coexist at the upper part of the culture tank and thus the possibility of explosion and the like is particularly concerned. In this respect, in the gas stirring fermentation device of the present invention, "electric energy of the stirrer to be an ignition source" can be excluded from among the factors causing explosion and the like because stirring of the culture liquid is achieved only by the flow action of the gas. This allows the gas stirring fermentation device of the present invention to reduce the possibility of explosion and the like. As will be described in detail below, the gas stirring fermentation device of the present invention can further reduce the concentration of oxygen discharged to the upper part of the culture tank due to remarkably high oxygen utilization efficiency. Therefore, the atmosphere in the upper part of the culture tank can be controlled so as not to fall within the combustion range of the flammable gas and thus the possibility of explosion and the like can be further reduced. Consequently, the gas stirring fermentation device of the present invention is particularly suitable for producing the flammable substance. In the present invention, the flammable substance is a substance being in a liquid state under 1 atmospheric pressure at 20°C and having a flash point of 70°C or less or a substrate having a lower limit value of a combustion limit of the substrate of 10% or less or having a lower limit value of a limiting oxygen concentration of 15% or less.

In the present invention, fermentation may be carried out using two-phase extraction fermentation in which hydrophobic substances are cultured together with organic substances and an organic layer including the hydrophobic substances may be recovered as gas. The organic substances used in the two-phase extraction fermentation are preferably organic solvents immiscible with water but is not limited to the organic solvents. The organic substance to be used is preferably organic solvents being harmless to the microorganisms used for fermentation production. Examples of the usable organic solvent include corn oil, dodecane, hexadecane, oleyl alcohol, butyl oleate, butyl phthalate, dodecanol, bis(2-ethylhexyl) phthalate, farnesene, isopropyl myristate, butanol, cyclohexane, n-tetradecane, and a mixed solvent thereof (Brennan TC et al., Alleviating monoterpene toxicity using a two-phase extractive fermentation for the bioproduction of jet fuel mixtures in Saccharomyces cerevisiae, Biotechnol Bioeng. 109, 2513-2522, 2012). The solvents, however, is not limited to these solvents. Alternatively, microorganisms that are resistant to organic solvents harmful to microorganisms such as toluene and benzene can also be used (Tadashi Honda et al., "Yuukiyoubaitaiseibiseibutuwo motiita hisuikeino sekaide monotukuri (Production in NonAqueous World Using Organic Solvent-Resistant Microorganisms)" Kankyou Biotechnology Gakkaishi (Environmental Biotechnology Journal) Vol. 6, No. 2 p.109-114, 2006).

The produced hydrophobic substance has moved to the organic layer and can be taken out from the fermentation medium by a dispensing operation. Alternatively, the hydrophobic substance may be taken out from the fermentation medium in combination with other type of an organic extract agent. Gas stripping may be carried out by passing gas such as air, nitrogen, or carbon dioxide through the fermentation medium, whereby a hydrophobic substance-containing gas phase may be formed. The hydrophobic substance product can be recovered from the hydrophobic substance-containing gas phase by using methods known in the technical field, for example, by using a chilled water trap in order to condense the hydrophobic substance or by scrubbing the gas phase with a solvent or may be recovered by combining unit operations such as cooling, absorption, adsorption, and membrane separation.

Hereinafter, each member included in the gas stirring fermentation device of the present invention will be described.

### -Culture tank-

The culture tank is not particularly limited as long as the culture tank can be provided with a gas supply pipe provided with a gas discharge part made of a sintered metal film described below and a draft tube. An appropriate culture tank may be selected depending on the properties of the chemical substances to be produced (specific gravity, presence or absence of flammability, and the like) and a production scale, the culture method (a batch culture method, a fed-batch culture method, a continuous culture method, and the like), and culture conditions (aerobic conditions, anaerobic conditions, and the like). The culture liquid will be described below.

### -Draft tube-

The draft tube is not particularly limited as long as the above gas lift action can be achieved and a known draft tube (also referred to as "gas lift pipe") may be used.

From the viewpoint of effectively achieving the gas lift action, the draft tube is generally located inside the culture tank so that the tube axis direction of the draft tube is perpendicular to the horizontal plane.

It has been known that the gas stirring fermentation device equipped with the draft tube enhances the mixing performance of the culture liquid due to an increase in the gas lift action when the gas superficial velocity in the draft tube is increased. In this respect, the gas stirring fermentation device of the present invention using the gas discharge part made of the sintered metal film has an larger increased value (ΔP_{M}) of the mixing performance (P_{M}) of the culture liquid obtained when the gas superficial velocity (V) is increased by a fixed value (ΔV) than the increased value of the conventional device using a coarse gas discharge part such as a single hole nozzle, which is described in Non-Patent Literature 1. In other words, in the gas stirring fermentation device of the present invention, as the gas superficial velocity in the draft tube is increased, excellent mixing performance can be achieved as compared with the mixing performance of the conventional device using a single hole nozzle or the like. The gas superficial velocity in the draft tube may be determined to achieve desired mixing performance and is preferably 0.001 m/s or more, more preferably 0.005 m/s or more, and further preferably 0.01 m/s. The upper limit of the gas superficial velocity is preferably high velocity from the viewpoint of mixing performance of the culture liquid and may be usually determined to be 0.2 m/s or less, 0.1 m/s or less, or 0.05 m/s or less.

At the time of the aerobic culture, oxygen-containing gas is discharged from the gas discharge part to stir the culture liquid and supply oxygen to the culture medium. In this respect, the inventors of the present invention have found that the gas stirring fermentation device allows the remarkably excellent oxygen supply performance as compared with the oxygen supply performance of the conventional device using the hole nozzle or the like to be achieved over a wide range of the gas superficial velocity in the draft tube, as long as the discharge gas linear velocity at the gas discharge part made of the sintered metal film described later is set to a certain value or less. Specifically, the gas stirring fermentation device of the present invention can achieve high oxygen utilization efficiency, which is even ten times higher than that of the conventional device using the single-hole nozzle, because the oxygen utilization efficiency (also referred to as oxygen dissolution efficiency or oxygen transfer efficiency) in accordance with a sodium sulfite method is more than 70% over a wide range of gas superficial velocity in the draft tube as long as the discharge gas linear velocity at the gas discharge part made of the sintered metal film is 0.04 m/s or less. Therefore, the concentration of oxygen discharged to the upper part of the culture tank can be reduced to a low level and the atmosphere in the upper part of the culture tank can be controlled so as not to fall within the combustion range of the flammable gas. This allows the possibility of explosion and the like to be further reduced.

### -Gas supply pipe-

The gas supply pipe has a function of supplying gas to the inside of the culture tank. In the gas stirring fermentation device of the present invention, the gas supply pipe is characterized in that the gas supply pipe is provided with a gas discharge part made of the sintered metal film. The details of the gas supplied to the inside of the culture tank will be described below.

The gas that has passed through the gas supply pipe is discharged from the gas discharge part made of the sintered metal film to the inside of the culture tank. The sintered metal film has a large number of pores and gas is discharged to the inside of the culture tank as fine bubbles depending on the diameter of the pores. As a result, the gas stirring fermentation device of the present invention can exhibit the excellent mixing performance as described above as compared with the mixing performance of the conventional device using the single hole nozzle because the residential time of the bubbles in the culture liquid becomes longer and the difference in the apparent concentration of the culture liquid between the inside and the outside of the draft tube becomes larger. In addition, the gas stirring fermentation device of the present invention generating fine air bubbles allows the value a in an oxygen transfer capacity coefficient K_{L}a (K_{L} represents a liquid film mass transfer coefficient and a represents a gas-liquid contact area per unit volume), which can be the indicator of the oxygen supply performance, to be remarkably high. As a result, the gas stirring fermentation device of the present invention allows the oxygen transfer capacity coefficient K_{L}a to be increased as compared with the oxygen transfer capacity coefficient of the conventional device using the single hole nozzle or the like and thus exhibits the excellent oxygen utilization efficiency as described above.

From the viewpoint of enhancing the mixing performance of the culture liquid and from the viewpoint of achieving the excellent oxygen supply performance also applicable to the aerobic culture with a high demanded oxygen amount, the average pore diameter of the sintered metal film is preferably 20 µm or less, more preferably 10 µm or less, and further preferably 8 µm or less, 6 µm or less, or 5 µm or less. The lower limit of the average pore diameter of the sintered metal film is not particularly limited and may usually be 0.1 µm or more, 0.5 µm or more, or 1 µm or more.

The sintered metal film can be produced by pressure molding metal powder having a uniform particle diameter distribution and sintering. By changing the average particle diameter of the metal powder, the sintering temperature, and the like, the average pore diameter of the sintered metal film to be obtained can be controlled. Examples of the material of the sintered metal film include nickel, stainless steel, inconel, titanium, and the like. From the viewpoints of mechanical strength, chemical resistance, thermal shock resistance, and the like, a sintered metal film made of stainless steel is preferable.

The gas discharge part made of the sintered metal film is aligned with the draft tube so that the culture liquid containing the gas discharged from the gas discharge part passes through the inside of the draft tube. Usually, the gas discharge part made of the sintered metal film is located near the lower opening of the draft tube.

In the gas stirring fermentation device of the present invention, even when the gas superficial velocity in the draft tube is the same, the mixing performance of the culture liquid may differ in some cases depending on the discharge gas linear velocity (Gas discharge amount [m³/s])/Surface area [m²] of sintered metal film) at the gas discharge part made of the sintered metal film. Specifically, in a range of the discharge gas linear velocity of 0.04 m/s or less, even when the discharge gas linear velocity changes, the mixing performance of the culture liquid is hardly affected if the gas superficial velocity in the draft tube is the same. In other words, in a range of the discharge gas linear velocity of 0.04 m/s or less, the influence of the gas superficial velocity may determine the mixing performance of the culture liquid. When the discharge gas linear velocity is more than 0.04 m/s, the mixing performance of the culture liquid gradually decreases if the gas superficial velocity in the draft tube is the same. Therefore, the discharge gas linear velocity at the gas discharge part made of the sintered metal film is preferably 0.04 m/s or less as long as the desired gas superficial velocity can be achieved. From the viewpoint of easily achieving the desired gas superficial velocity, the lower limit of the discharge gas linear velocity is preferably 0.005 m/s or more, more preferably 0.01 m/s or more, 0.02 m/s or more, or 0.03 m/s or more,

From the viewpoint of the oxygen supply performance, the discharge gas linear velocity at the gas discharge part made of the sintered metal film is preferably 0.04 m/s or less. When the discharge gas linear velocity is 0.04 m/s or less, the oxygen utilization efficiency of more than 70% can be achieved over a wide range of gas superficial velocity in the draft tube. The inventors of the present invention have found that the oxygen utilization efficiency decreases as the discharge gas linear velocity increases in the range where the discharge gas linear velocity is more than 0.04 m/s.

From the viewpoint of achieving the gas stirring fermentation device having the excellent mixing performance of the culture liquid and applicable to the aerobic culture with the high demanded oxygen amount, the discharge gas linear velocity at the gas discharge part made of the sintered metal film is preferably in a range of 0.04 m/s or less, more preferably in a range of 0.005 m/s to 0.04 m/s, further preferably in a range of 0.01 m/s to 0.04 m/s, in a range of 0.02 m/s to 0.04 m/s, or in a range of 0.03 m/s to 0.04 m/s.

The shape and size of the gas discharge part made of the sintered metal film are not particularly limited as long as the suitable discharge gas linear velocity can be achieved. In order to achieve the suitable discharge gas linear velocity and to achieve the desired gas superficial velocity, a plurality of gas discharge parts made of the sintered metal films may be provided.

The gas stirring fermentation device of the present invention may include other elements necessary for producing the chemical substance by the fermentation method. Examples of such other elements include a culture liquid supply pipe supplying the culture medium to the inside of the culture tank, a base compound supply pipe supplying a basic compound for controlling pH to the inside of the culture tank, a chemical substance recovery pipe for recovering the produced chemical substance, a gas discharge pipe for taking out gas at the upper part of the culture tank to the outside, a temperature regulator for controlling the temperature of the culture tank, and the like. Known elements commonly used in fermentation devices may be used for these elements.

Hereinafter, the embodiment of the gas stirring fermentation device of the present invention will be described with reference to the drawings.

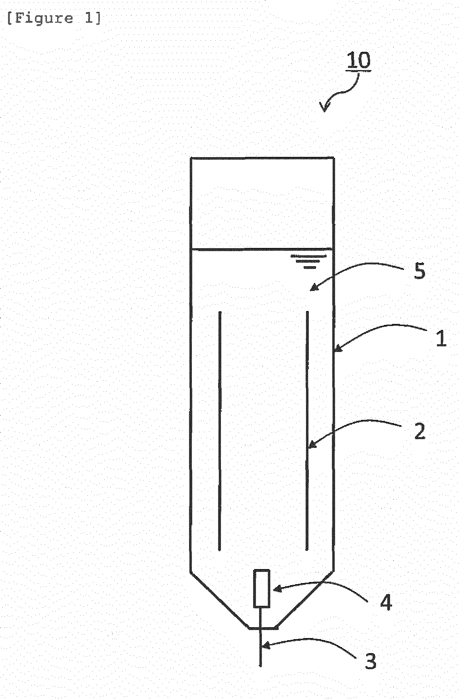

In FIG. 1, a schematic view illustrating a gas stirring fermentation device according to an embodiment of the present invention is illustrated. In FIG. 1, the gas stirring fermentation device 10 includes the culture tank 1, the draft tube 2 located inside the culture tank, and the gas supply pipe 3 supplying gas to the inside of the culture tank, in which the gas supply pipe 3 is provided with the gas discharge part 4 made of the sintered metal film. The gas stirring fermentation device 10 can stir the culture liquid 5 in the culture tank with the gas discharged from the gas discharge part. In FIG. 1, an embodiment having one gas discharge part made of the sintered metal film is illustrated. However, in order to achieve the suitable discharge gas linear velocity and in order to achieve the desired gas superficial velocity, a plurality of gas discharge parts made of the metal films may be provided.

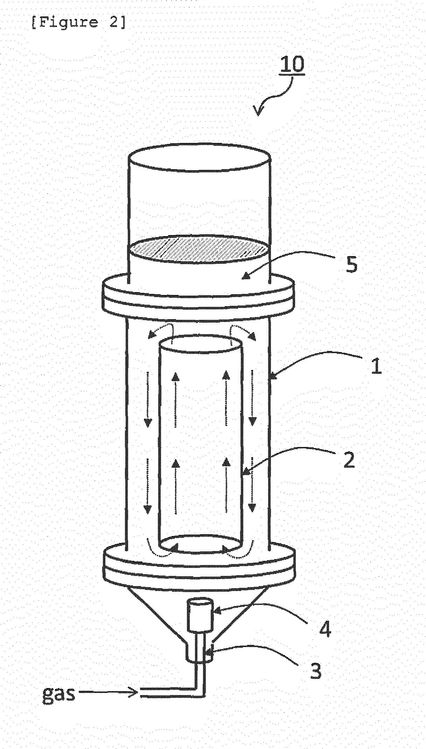

In FIG. 2, a schematic view for illustrating the circulation flow of the culture liquid in the gas stirring fermentation device according to an embodiment of the present invention is illustrated. The meaning of each sign is the same as in FIG. 1. In the gas stirring fermentation device 10, the gas that has passed through the gas supply pipe 3 is discharged from the gas discharge part 4 made of the sintered metal film to the inside of the culture tank 1. The sintered metal film has a large number of pores and the gas is discharged to the inside of the culture tank as fine bubbles depending on the diameter of the pores. The culture liquid containing more bubbles and having a low apparent density flows inside the draft tube 2 upward. Outside the draft tube, the culture liquid having less bubble content and a high apparent density flows downward. In this way, the upward flow of the culture liquid occurs inside the draft tube and the downward flow of the culture liquid occurs outside the draft tube. Therefore, the circulation flow of the culture liquid occurs inside the culture tank and the culture liquid is stirred.

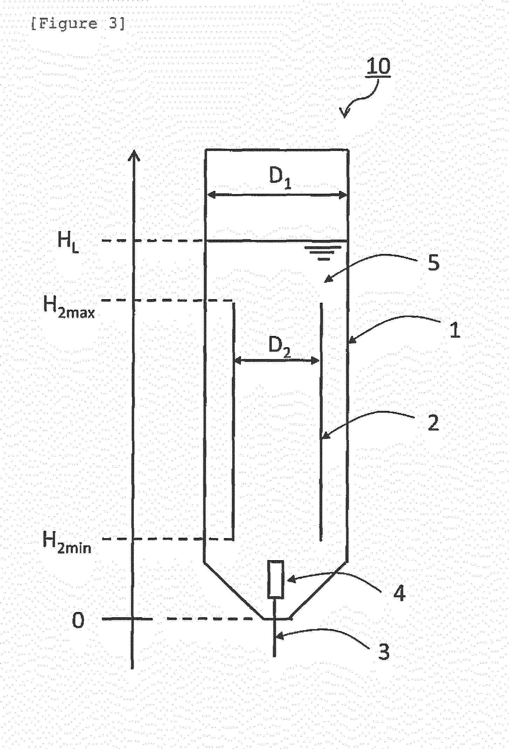

In FIG. 3, a schematic view for illustrating suitable sizes and arrangements of the gas stirring fermentation device of the present invention is illustrated. The meaning of each sign is the same as in FIG. 1.

As shown in FIG. 3, from the viewpoint of further enhancing the mixing performance of the culture liquid, the gas stirring fermentation device of the present invention preferably satisfies the relation of 0.7<D₂/D₁ where D₁ is the inner diameter of the culture tank 1 and D₂ is the inner diameter of the draft tube 2. From the viewpoint of the mixing performance of the culture liquid, the ratio D₂/D₁ is more preferably 0.75 or more and further preferably 0.8 or more, 0.82 or more, 0.84 or more, 0.86 or more, or 0.88 or more. From the viewpoint of the mixing performance of the culture liquid, the upper limit of the ratio D₂/D₁ is preferably 0.98 or less, more preferably 0.96 or less, further preferably 0.94 or less or 0.92 or less. From the viewpoint of achieving the smooth circulation flow of the culture liquid, the draft tube 2 is preferably located coaxially with the culture tank 1.

When the inner diameter of the culture tank 1 is D₁, the inner diameter of the draft tube 2 is D₂, the liquid level height of the culture liquid 5 from the bottom of the culture tank 1 is H_{L}, the height of the uppermost part of the draft tube 2 from the bottom of the culture tank 1 is H₂ₘₐₓ, and the height of the lowermost part of the draft tube 2 from the bottom of the culture tank 1 is H₂ₘᵢₙ, the gas stirring fermentation device of the present invention preferably satisfies at least one of the following conditions (i) and (ii).
(i): 1≤(H_{L}-H₂ₘₐₓ)/D₁
(ii): H₂ₘᵢₙ/D₂≤2

For the condition (i), the ratio (H_{L}-H₂ₘₐₓ)/D₁ is more preferably 1.5 or more and further preferably 2 or more from the viewpoint of enhancing the mixing performance of the culture liquid. The upper limit of the ratio (H_{L}-H₂ₘₐₓ)/D₁ is not particularly limited as long as the smooth circulation flow of the culture liquid can be achieved and may be usually, for example, 5 or less or 4 or less.

For the condition (ii), the ratio H₂ₘᵢₙ/D₂ is more preferably 1.5 or less, further preferably 1 or less, 0.9 or less, 0.8 or less, 0.7 or less, or 0.6 or less from the viewpoint of enhancing the mixing performance of the culture liquid. The lower limit of the ratio H₂ₘᵢₙ/D₂ is not particularly limited as long as the smooth circulation flow of the culture liquid can be achieved and can be usually, for example, 0.1 or more, 0.2 or more, 0.3 or more, or 0.4 or more.

The gas stirring fermentation device of the present invention allows stirring of the culture liquid to be achieved only by the flow action of the gas and the mechanical stirrer to be substantially eliminated. On the other hand, the gas stirring fermentation device of the present invention allows the oxygen utilization efficiency to be extremely high and the concentration of oxygen discharged to the upper part of the culture tank to be kept low. Therefore, the atmosphere in the upper part of the culture tank can be controlled so as not to fall within the combustion range and, even when a mechanical stirrer is used, the possibility of explosion and the like can be reduced.

### [Method for producing chemical substance]

The method for producing the chemical substance of the present invention includes:
a step of discharging gas from a gas discharge part made of a sintered metal film into a culture liquid including microorganisms having ability to produce the chemical substance and passing the culture liquid including the discharged gas through the inside of a draft tube to stir the culture liquid; and
a step of culturing the microorganisms to produce the chemical substance.

The chemical substance being the production target, the gas discharge part made of the sintered metal film, and the draft tube are as described in [Gas stirring fermentation device] described above. The method for producing the chemical substance of the present invention is characterized in that the culture liquid is stirred by the gas lift action of the gas discharged from the gas discharge part made of the sintered metal film and can achieve the excellent mixing performance of the culture liquid, which cannot be achieved by a conventional gas stirring fermentation method. When oxygen-containing gas is used as the gas to be discharged from the gas discharge part, excellent oxygen supply performance can be achieved to the extent applicable to the aerobic culture with the high demanded oxygen amount. Advantageous effects described for the gas stirring fermentation device of the present invention are similarly applied to the method for producing the chemical substance of the present invention.

In the present invention, the "microorganisms having ability to produce a chemical substance" includes both 1) microorganisms that can inherently produce the chemical substance and 2) microorganisms that do not originally have or do not substantially have the ability to produce the chemical substance but have been genetically modified to introduce a chemical substance production gene and have acquired capability of producing the chemical substance afterward. With regard to the microorganisms that can produce chemical substances, various kinds of microorganisms are known depending on the kind of the chemical substances and these known microorganisms may be widely used in the present invention. As long as the microorganisms have the ability to produce the chemical substances, the present invention can be widely applied to microorganisms to be developed in the future.

Suitable microorganisms are exemplified below for embodiments in which the chemical substance being the production target is isoprenoid compounds.

Examples of Gram-positive bacteria include Bacillus bacteria, Listeria bacteria, Staphylococcus bacteria, Streptococcus bacteria, Enterococcus bacteria, Clostridium bacteria, Corynebacterium bacteria, Streptomyces bacteria, and the like. Bacillus bacteria and Corynebacterium bacteria are preferable.

Examples of Bacillus bacteria include *Bacillus subtilis, Bacillus anthracis, Bacillus cereus* and the like, and *Bacillus subtilis* is more preferable.

Examples of Corynebacterium bacteria include *Corynebacterium glutamicum, Corynebacterium efficiens,* and *Corynebacterium callunae* and the like, and *Corynebacterium glutamicum* is more preferable.

Examples of Gram-negative bacteria include Escherichia bacteria, Pantoea bacteria, Salmonella bacteria, Vi.vrio bacteria, Serratia bacteria, Enterobacter bacteria and the like. Escherichia bacteria, Pantoea bacteria, and Enterobacter bacteria are preferable.

As Escherichia bacteria, *Escherichia coli* is preferable.

Examples of Pantoea bacteria include *Pantoea ananatis, Pantoea stewartii, Pantoea agglomerans, Pantoea citrea* and the like, and *Pantoea ananatis* and *Pantoea citrea* are preferable. As Pantoea bacteria, strains exemplified in European Patent Application Laid-open No. 0952221 may be used. Examples of the representative strains of Pantoea bacteria include Pantoea *ananatis* AJ13355 strain (FERM BP-6614) and *Pantoea ananatis* AJ13356 strain (FERM BP-6615) disclosed in European Patent Application Laid-open No. 0952221.

Examples of Enterobacter bacteria include Enterobacter *agglomerans, Enterobacter aerogenes* and the like, and Enterobacter aerogenes is preferable. As Enterobacter bacteria, a strain exemplified in European Patent Application Laid-open No. 0952221 may also be used. Examples of the representative strains of Enterobacter bacteria include *Enterobacter agglomerans* ATCC12287 strain, *Enterobacter aerogenes* ATCC13048 strain, *Enterobacter aerogenes* NBRC12010 strain (Biotechnol Bioeng. 2007 Mar 27; 98 (2): 340-348), *Enterobacter aerogenes* AJ110637 (FERM BP-10955) strain and the like. *Enterobacter aerogenes* AJ110637 strain was deposited on Aug. 22, 2007 at National Institute of Advanced Industrial Science and Technology, International Patent Organism Depositary Center (Central 6, 1-1, Higashi 1-chome, Tsukuba-shi, Ibaraki-ken, Japan (Postal Code 305-8566)) as a deposition number of FERM P-21348 and transferred to an international deposit under Budapest Treaty on March 13, 2008 to provide a receipt number of FERM BP-10955.

Examples of fungi include microorganisms of genus Saccharomyces, Schizosaccharomyces, Yarrowia, Trichoderma, Aspergillus, Fusarium, Mucor and the like. The microorganisms of genus Saccharomyces, Schizosaccharomyces, Yarrowia, and Trichoderma are preferable.

Examples of the microorganisms of genus Saccharomyces include *Saccharomyces carlsbergensis, Saccharomyces cerevisiae, Saccharomyces diastaticus, Saccharomyces douglasii, Saccharomyces kluyveri, Saccharomyces norbensis,* and *Saccharomyces oviformis* and *Saccharomyces cerevisiae* is preferable.

As the microorganisms of genus Schizosaccharomyces, *Schizosaccharomyces pombe* is preferable.

As the microorganism of genus Yarrowia, *Yarrowia lipolytica* is preferable.

Examples of microorganisms of the genus Trichoderma include *Trichoderma harzianum, Trichoderma koningii, Trichoderma longibrachiatum,* Trichoderma reesei, and *Trichoderma viride. Trichoderma reesei* is preferable.

To the microorganisms that do not inherently have or do not substantially have the ability to produce the isoprenoid compounds, the ability to produce the isoprenoid compounds can be provide by introducing a gene encoding an isoprenoid compound synthesis enzyme that is an enzyme of dimethylallyl diphosphate supply pathway with an expression vector or introducing the gene on a chromosome by gene recombination.

Examples of the dimethylallyl diphosphate supply pathway includes the methyl erythritol phosphate (MEP) pathway and the mevalonic acid (MVA) pathway.

The methyl erythritol phosphate (MEP) pathway is a biosynthetic pathway of isopentenyl diphosphate (IPP) and dimethylallyl diphosphate (DMAPP) (Nat. Prod. Rep. 16 (5): 565-574 1999). The methyl erythritol phosphate (MEP) pathway is also referred to as the MEP pathway, a DXP pathway, a DOXP pathway, and an MEP/DOXP pathway because methyl erythritol phosphate (MEP) and 1-deoxy-D-xylulose-5-phosphate (DXP or DOXP) are biosynthesized as metabolic intermediates.

Examples of enzymes involved in the methyl erythritol phosphate (MEP) pathway include 1-deoxy-D-xylulose-5-phosphate synthase (EC: 2.2.1.7, Example 1, Dxs, ACCESSION ID NP_414954; Example 2, AT3G21500, ACCESSION ID NP_566686; Example 3, AT4G15560, ACCESSION ID NP_193291; Example 4, AT5G11380, ACCESSION ID NP_001078570), 1-deoxy-D-xylulose-5-phosphate reductoisomerase (EC: 1.1.1.267; Example 1, Dxr, ACCESSION ID NP_414715; Example 2, AT5G62790, ACCESSION ID NP_001190600), 4-diphosphocytidyl-2-C-methyl-D-erythritol synthase (EC: 2.7.7.60; Example 1, IspD, ACCESSION ID NP_417227; Example 2, AT2G02500, ACCESSION ID NP_565286), 4-diphosphocytidyl-2-C-methyl-D-erythritol kinase (EC: 2.7.1.148; Example 1, IspE, ACCESSION ID NP_415726; Example 2, AT2G26930, ACCESSION ID NP_180261), 2-C-methyl-D-erythritol-2,4-cyclodiphosphate synthase (EC: 4.6.1.12; Example 1, IspF, ACCESSION ID NP_417226; Example 2, AT1G63970, ACCESSION ID NP_564819), 1-hydroxy-2-methyl-2-(E)-butenyl-4-diphosphate synthase (EC: 1.17.7.1; Example 1, IspG, ACCESSION ID NP_417010; Example 2, AT5G60600, ACCESSION ID NP_001119467), and 4-hydroxy-3-methyl-2-butenyl diphosphate reductase (EC: 1.17.1.2; Example 1, IspH, ACCESSION ID NP_414570; Example 2, AT4G34350, ACCESSION ID NP_567965).

The mevalonic acid (MVA) pathway is a biosynthetic pathway in which isopentenyl diphosphate and dimethylallyl diphosphate being the starting synthesis substance of the isoprenoids are synthesized from acetyl CoA. Examples of enzymes involved in the mevalonic acid (MVA) pathway include mevalonate kinase (EC: 2.7.1.36; Example 1, Erg12p, ACCESSION ID NP_013935; Example 2, AT5G27450, ACCESSION ID NP_001190411), phosphomevalonate kinase (EC: 2.7.4.2; Example 1, Erg8p, ACCESSION ID NP_013947; Example 2, AT1G31910, ACCESSION ID NP_001185124), diphosphomevalonate decarboxylase (EC: 4.1.1.33; Example 1, Mvdlp, ACCESSION ID NP_014441; Example 2, AT2G38700, ACCESSION ID NP_181404; Example 3, AT3G54250, ACCESSION ID NP_566995), acetyl-CoA-C-acetyltransferase (EC: 2.3.1.9; Example 1, Erg10p, ACCESSION ID NP_015297; Example 2, AT5G47720, ACCESSION ID NP_001032028; Example 3, AT5G48230, ACCESSION ID NP_568694), hydroxymethylglutaryl-CoA synthase (EC: 2.3.3.10; Example 1, Erg13p, ACCESSION ID NP-013580; Example 2, AT4G11820, ACCESSION ID NP_192919; Example 3, MvaS, ACCESSION ID AAG02438), hydroxymethylglutaryl-CoA reductase (EC: 1.1.1.34; Example 1, Hmg2p, ACCESSION ID NP_013555; Example 2, Hmglp, ACCESSION ID NP_013636; Example 3, AT1G76490, ACCESSION ID NP_177775; Example 4, AT2G17370, ACCESSION ID NP_179329, EC: 1.1.1.88, Example 5, MvaA, ACCESSION ID P 13702), acetyl-CoA-C-acetyltransferase/hydroxymethylglutaryl-CoA reductase (EC: 2.3.1.9/1.1.1.34, Example, MvaE, ACCESSION ID AAG02439). In the expression vector, the gene coding one or more enzymes involved in the mevalonic acid (MVA) pathway (for example, phosphomevalonate kinase, diphosphomevalonate decarboxylase, acetyl-CoA-C-acetyltransferase/hydroxymethyl glutaryl-CoA reductase, hydroxymethylglutaryl-CoA synthase) may be placed under the control of the growth promoter reverse-dependent promoter.

The isoprenoid compound-producing microorganism may further have an enhanced pathway for synthesizing dimethylallyl diphosphate (DMAPP) being an isoprenoid compound material (for example, a substrate of isoprene synthase). For such enhancement, an expression vector of isopentenyl diphosphate delta isomerase having the ability to convert isopentenyl diphosphate (IPP) to dimethylallyl diphosphate (DMAPP) is introduced into the isoprenoid compound-producing microorganism. The expression vectors for one or more enzymes involved in the mevalonic acid pathway and/or the methylerythritol phosphate pathways associated with the production of IPP and/or DMAPP may also be introduced into the isoprenoid compound-producing microorganism. The expression vector of such enzymes may be an integrative vector or may be a non-integrative vector. The expression vectors for such enzymes further express several enzymes (for example, one enzyme, two enzymes, three enzymes, or four or more enzymes) involved in the mevalonic acid pathway and/or the methylerythritol phosphate pathway together or individually. For example, the expression vector may be an expression vector of polycistronic mRNA. The origin of one or more enzymes involved in the mevalonic acid pathway and/or the methylerythritol phosphate pathway may be homologous or heterologous to the host. When the origin of the enzyme involved in the mevalonic acid pathway and/or the methylerythritol phosphate pathway is heterologous to the host, for example, the host is bacteria (for example, *Escherichia coli*) as described above and the enzyme involved in the mevalonic acid pathway may be originated from fungi (for example, *Saccharomyces cerevisiae*)*.* When the host intrinsically produces the enzyme involved in the methylerythritol phosphate pathway, the expression vector introduced into the host may express the enzyme involved in the mevalonic acid pathway.

When the chemical substance being the production target is isoprene, the isoprene synthase is preferably intracellularly enhanced in addition to the enzyme of the MEP pathway or the MVA pathway. Examples of the isoprene synthase include isoprene synthases originated from *Pueraria* montana *var. lobata,* Populus *alba x Populus tremula, Mucuna bracteata,* Salix, *Robinia pseudoacacia,* Wisterria, *Eucalyptus globulus,* and *Melaleuca alterniflora* (refer to, for example, Evolution 67 (4), 1026-1040 (2013)). The isoprenoid compound synthase expression vector may be an integrative vector or may be a non-integrative vector.

When the chemical substance being the production target is a carbon-containing substance such as hydrocarbon, the culture liquid preferably includes a carbon source. Examples of the carbon source include carbohydrates such as monosaccharides, disaccharides, oligosaccharides and polysaccharides; invert sugar obtained by hydrolyzing sucrose; glycerol; substances having one carbon atom such as methanol, formaldehyde, formate, carbon monoxide, and carbon dioxide; oils such as corn oil, palm oil, and soybean oil; acetates, animal fats; animal oils; fatty acids such as saturated fatty acids and unsaturated fatty acids; lipids; phospholipids; glycerolipids; glycerin fatty acid esters such as monoglycerides, diglycerides, and triglycerides; polypeptides such as microbial proteins and vegetable proteins; renewable carbon sources such as decomposed biomass carbon sources; yeast extracts; and combinations thereof. When the chemical substance being the production target is hydrogen, the culture liquid preferably includes a hydrogen-containing material in the materials exemplified as the carbon sources. The culture liquid further includes nitrogen sources, inorganic ions, and, if necessary, other organic trace components. As such nitrogen sources, inorganic ions, and other organic trace components, any conventionally known components may be used.

When the isoprenoid compound is isoprene, the method of the present invention can be carried out in a system including a liquid phase and a gas phase. As such a system, a closed system, for example, a reactor such as a fermenter and a fermentation tank can be used in order to avoid disappearance of the produced isoprene due to the diffusion of the isoprene. As the liquid phase, a culture medium including isoprene-producing microorganisms can be used. The gas phase is the space above the liquid phase in the system, which is also referred to as a head space, and includes the fermentation gas. Isoprene has a boiling point of 34°C at standard atmospheric pressure, low solubility in water (solubility in water: 0.6 g/L), and a vapor pressure at 20°C of 60.8 kPa (for example, Brandes et al., Physikalish Technische Bundesanstalt (PTB), 2008) and thus isoprene produced in the liquid phase may easily transfer to the gas phase, when the isoprene-producing microorganisms are cultured in a liquid phase under a temperature condition of 34°C or higher. Therefore, when such a system is used, the isoprene produced in the liquid phase can be recovered from the gas phase. In addition, the isoprene produced in the liquid phase can easily move into the gas phase, the isoprene production reaction by the isoprene-producing microorganisms (enzyme reaction by isoprene synthase) in the liquid phase is always inclined to the isoprene production side.

When the method of the present invention is carried out in the system including the liquid phase and the gas phase, the oxygen concentration in the gas phase is desirably controlled. From the viewpoint of avoiding explosion, the oxygen concentration in the gas phase is required to be controlled because isoprene has an explosive limit of 1.0% to 9.7% (w/w) (for example, Brandes et al., Physikalish Technische Bundesanstalt (PTB), 2008), the property of being easy to explode, and the explosion range that fluctuates depending on the mixing ratio with oxygen in the gas phase (refer to US 8420360 B2, FIG. 24).

The control of the oxygen concentration in the gas phase can be carried out by supplying gas in which the oxygen concentration is controlled into the system. The gas supplied into the system may include gas components such as nitrogen, carbon dioxide, and argon in addition to oxygen. More specifically, the oxygen concentration in the gas phase can be controlled by adding inert gas so that the oxygen concentration is equal to or lower than the limit oxygen concentration of the gas having the explosion range. As the gas components other than oxygen, inert gas is preferable. Preferably, the gas in which the oxygen concentration is controlled is supplied into the liquid phase, whereby the oxygen concentration in the gas phase is indirectly controlled. This is because the oxygen concentration in the gas phase can be controlled by controlling the dissolved oxygen concentration in the liquid phase as described below.

Oxygen in the gas supplied into the liquid phase dissolves in the liquid phase and eventually reaches the saturated concentration. On the other hand, in the system where microorganisms exist, the dissolved oxygen in the liquid phase is consumed by the metabolic activity of the cultured microorganisms and, as a result, the dissolved oxygen concentration falls below the saturated concentration. The oxygen in the gas phase or the oxygen in the newly supplied gas can be transferred to the liquid phase by gas-liquid equilibrium in the liquid phase containing oxygen at the saturated concentration or lower. In other words, the oxygen concentration in the gas phase decreases depending on the oxygen consumption rate of the microorganism. The oxygen concentration in the gas phase can also be controlled by controlling the oxygen consumption rate of the cultured microorganism.

For example, the concentration in the gas phase can be set to 9% (v/v) or less (for example, 5% (v/v) or less, 0.8 (v/v) or less, 0.6% (v/v) or less, 0.5% (v/v) or less, 0.4% (v/v) or less, 0.3% (v/v) or less, 0.2% (v/v) or less, or 0.1% (v/v) or less) or substantially 0% (v/v) by increasing the metabolism rate of the carbon source of the microorganisms to increase the oxygen consumption rate. Alternatively, the control of the oxygen concentration can be achieved by setting the oxygen concentration in the gas to be initially supplied low. Therefore, the oxygen concentration in the gas phase can be set in consideration of the oxygen consumption rate by the microorganisms in the liquid phase and the oxygen concentration in the gas to be supplied.

The culture can be carried out using a liquid culture medium. In the culture, microorganisms cultured in a solid culture medium such as an agar culture medium or the like may be directly inoculated into the liquid culture medium or microorganisms cultured by seed culture in a liquid culture medium is inoculated into the liquid culture medium for the main culture. In other words, the culture may be separately carried out in the seed culture and the main culture. In this case, the culture conditions of the seed culture and the main culture may be the same or different. The amount of the microorganisms included in the culture medium at the start of the culture is not particularly limited. For example, a seed culture liquid having an OD660 of 4 to 8 may be added to the culture medium for the main culture at the time of the start of culture from 0.1% by mass to 30% by mass and preferably from 1% by mass to 10% by mass.

The culture can be carried out by batch culture, fed-batch culture, continuous culture, or a combination thereof. The culture medium at the time of the start of culture is also referred to as an "initial medium". The culture medium to be fed to the culture system (fermenter) in the fed-batch culture or the continuous culture is referred to as a "fed-batch medium". The feeding of the fed-batch culture medium to the culture system in the fed-batch culture or the continuous culture is referred to as "fed-batch". When the culture is separately carried out in the seed culture and the main culture, for example, both of the seed culture and the main culture may be carried out by the batch culture. In addition, for example, the seed culture may be carried out by the batch culture and the main culture may be carried out by the fed-batch culture or the continuous culture.

The culture may be carried out under aerobic conditions, microaerobic conditions, or anaerobic conditions. The culture is preferably carried out under the microaerobic or the anaerobic conditions. The aerobic condition means that the dissolved oxygen concentration in the liquid culture medium is 0.33 ppm, which is the detection limit by an oxygen membrane electrode, or more and may be preferably 1.5 ppm or more. The microaerobic condition means that oxygen is supplied to the culture system but the dissolved oxygen concentration in the liquid culture medium is less than 0.33 ppm. The anaerobic condition means a condition in which oxygen is not supplied to the culture system. The culture may be carried out under the conditions selected above during the entire period or may be carried out under the conditions selected above only for a limited period of time. Specifically, "culture is carried out under the aerobic conditions" means that the culture is carried out under the aerobic conditions for at least a part of the period in the entire period of the culture. "Culture is carried out under the microaerobic conditions" means that the culture is carried out under the microaerobic conditions for at least a part of the period in the entire period of the culture. "Culture is carried out under the anaerobic conditions" means that the culture is carried out under the anaerobic conditions for at least a part of the period in the entire period of the culture. "A part of the period" may be, for example, a period of 50% or more, 70% or more, 80% or more, 90% or more, 95% or more, or 99% or more of the entire period of the culture. In addition, "the entire period of the culture" may mean the entire period of the main culture when the culture is separately carried out in the seed culture and the main culture. The microaerobic conditions or anaerobic conditions can be achieved due to a decrease in the dissolved oxygen concentration in the liquid culture medium by means such as decreasing the aeration amount and the stirring speed, culturing without aeration by sealing the container, and aerating an inert gas containing carbonic acid gas.

The pH of the culture medium may be, for example, a pH of 3 to 10 and preferably a pH of 4.0 to 9.5. During the culture, the pH of the culture medium can be adjusted, if necessary. The pH of the culture medium can be controlled using various alkaline or acidic substances such as ammonia gas, aqueous ammonia, sodium carbonate, sodium bicarbonate, potassium carbonate, potassium bicarbonate, magnesium carbonate, sodium hydroxide, calcium hydroxide, and magnesium hydroxide.

The culture medium may include carbonate ion, bicarbonate ion, carbonic acid gas, or a combination thereof. For example, these components may be supplied by the metabolism of microorganisms or may be supplied from a carbonate salt and/or a bicarbonate salt used for pH control. These components can also be supplied by separately adding carbonic acid, bicarbonic acid, a salt thereof, or carbonic acid gas, if necessary. Specific examples of the salt of carbonic acid or bicarbonic acid include calcium carbonate, magnesium carbonate, ammonium carbonate, sodium carbonate, potassium carbonate, ammonium bicarbonate, sodium bicarbonate, and potassium bicarbonate. Carbonate ion and/or bicarbonate ion may be added at a concentration of 0.001 M to 5 M, preferably 0.1 M to 3 M, and further preferably 1 M to 2 M. When carbonic acid gas is included, for example, carbonic acid gas may be contained in an amount of 50 mg to 25 g, preferably 100 mg to 15 g, and further preferably 150 mg to 10 g per 1 L of the solution.

The culture temperature may be, for example, 20°C to 45°C and preferably 25°C to 37°C. The culture period may be, for example, 10 hours to 120 hours. The culture may be continued, for example, until the carbon source in the culture medium is consumed or until the activity of the microorganisms disappears.

In the method of the present invention, gas is discharged from the gas discharge part made of the sintered metal film and the culture liquid including the discharged gas is passed through the inside of the draft tube to stir the culture liquid. The gas-containing culture liquid has a lower apparent density and usually flows upward perpendicular to the horizontal plane. Therefore, in the method of the present invention, the draft tube is preferably provided so that the tube axis direction thereof is perpendicular to the horizontal plane so as to pass the culture liquid having a lower apparent density containing gas through the inside of the draft tube and thus the gas is preferably discharged from the gas discharge part provided in the vicinity of the lower opening part. The stirring mechanism of the culture liquid by the gas lift action is as described above.

In the present invention, the gas discharged from the gas discharge part may be determined depending on the culture conditions (aerobic condition, anaerobic condition, and the like) of the microorganisms. When the microorganisms are cultured under the aerobic conditions, the gas discharged from the gas discharge part is preferably oxygen-containing gas. The oxygen-containing gas is not particularly limited as long as the oxygen-containing gas has a sufficient oxygen concentration for culturing the microorganisms under the aerobic conditions and examples of the oxygen-containing gas include air, oxygen-enriched air, pure oxygen, and mixed gas made of mixing the oxygen-containing gas and an inert gas (nitrogen and the like). When the microorganisms are cultured under the anaerobic conditions, the gas discharged from the gas discharge part is not particularly limited as long as the gas does not substantially contain oxygen and examples the gas include nitrogen, carbon dioxide, hydrogen, methane, carbon monoxide, and mixed gas thereof

A suitable range of the discharge gas linear velocity at the gas discharge part made of the sintered metal film and the gas superficial velocity in the draft tube is as described in [Gas stirring fermentation device]. In one suitable embodiment, the discharge gas linear velocity at the gas discharge part made of the sintered metal film is preferably 0.04 m/s or less.

The method of the present invention may further include recovering the chemical substance. The chemical substance has low solubility in the culture liquid and can be easily separated and recovered. The chemical substance may be recovered as liquids or the chemical substance may be recovered as gases depending on the type of the chemical substance. In order to obtain the chemical substance having high purity, purification/isolation treatment may be carried out in accordance with known methods.

### [Example]

### Preparation Example 1: Construction of microaerobic induction type isoprenoid compound-producing microorganism (SWITCH-Plld/IspSM), Phosphate Deficiency induction type isoprenoid compound-producing microorganisms (SWITCH-PphoC/IspSM and SWITCH-PpstS/IspSM), and arabinose induction type isoprenoid compound-producing microorganism (SWITCH-Para/IspSM)

### 1-1) Construction of pMW-Para-mvaES-Ttrp

### 1-1-1) Chemical synthesis of mvaE gene originated from Enterococcus faecalis

The base sequence and amino acid sequence of mvaE originated from *Enterococcus faecalis* encoding acetyl-CoA acetyltransferase and hydroxymethlglutaryl-CoAreductase have been already known (ACCESSION No. of the base sequence: AF290092.1, (1479..3890), ACCESSION No. of amino acid sequence: AAG02439) (J. Bacteriol. 182 (15), 4319-4327 (2000)). The amino acid sequence of the mvaE protein and the base sequence of the gene originated from Enterococcus *faecalis* are shown in SEQ ID NO: 5 and SEQ ID NO: 6, respectively. In order to effectively express the mvaE gene in E. coli, the mvaE gene optimized for frequency in use of the codon of E. coli was designed and named EFmvaE. This base sequence is shown in SEQ ID NO: 7. The mvaE gene was chemically synthesized and thereafter cloned into pUC57 (manufactured by GenScript Co., Ltd) and the obtained plasmid was named pUC57-EFmvaE.

### 1-1-2) Chemical synthesis of mvaS gene originated from Enterococcus faecalis

The base sequence and the amino acid sequence of mvaS originated from *Enterococcus faecalis* encoding hydroxymethylglutaryl-CoA synthase have been already known (ACCESSION No. of the base sequence: AF290092.1, complement (142..1293), ACCESSION No. of the amino acid sequence: AAG02438) (J. Bacteriol. 182 (15), 4319-4327 (2000)). The amino acid sequence of the mvaS protein and the base sequence of the gene originated from *Enterococcus faecalis* are shown in SEQ ID NO: 8 and SEQ ID NO: 9, respectively. In order to effectively express the mvaS gene in *E. coli,* the mvaS gene optimized for frequency in use of the codon of *E. coli* was designed and named EFmvaS. This base sequence is shown in SEQ ID NO: 10. The mvaS gene was chemically synthesized and thereafter cloned into pUC57 (manufactured by GenScript Co., Ltd) and the obtained plasmid was named pUC57-EFmvaS.

### 1-1-3) Construction of arabinose induction type mvaES expression vector

The arabinose induction type mevalonic acid pathway upstream gene expression vector was constructed by the following procedure. Using a plasmid pKD46 as a template, PCR fragment including Para formed of araC and araBAD promoter sequence originated from *E. coli* was obtained by PCR using synthetic oligonucleotides shown in SEQ ID NO: 11 and SEQ ID NO: 12 as primers. Using the plasmid pUC57-EFmvaE as a template, a PCR fragment including the EFmvaE gene was obtained by PCR using the synthetic oligonucleotides shown in SEQ ID NO: 13 and SEQ ID NO: 14 as primers. Using the plasmid pUC57-EFmvaS as a template, a PCR fragment including the EFmvaS gene was obtained by PCR using the synthetic oligonucleotides shown in SEQ ID NO: 15 and SEQ ID NO: 16 as primers. Using a plasmid pSTV-Ptac-Ttrp as a template, a PCR fragment including the Ttrp sequence was obtained by PCR and synthetic oligonucleotides shown in SEQ ID NO: 17 and SEQ ID NO: 18 as primers. As PCR for obtaining these four PCR fragments, Prime Star polymerase (manufactured by Takara Bio Inc.) was used. The reaction solution was prepared according to the composition attached to the kit and subjected to 30 cycles of reaction at 98°C for 10 seconds, 55°C for 5 seconds, and 72°C for 1 minute/kb. PCRs were carried out using the PCR product containing the purified Para and the PCR product containing the EFmvaE gene as templates and the synthetic oligonucleotides shown in SEQ ID NO: 11 and SEQ ID NO: 14 as primers and using the PCR product containing the purified EFmvaS gene and the PCR product containing Ttrp as templates and the synthetic oligonucleotides shown in SEQ ID NO: 15 and SEQ ID NO: 18 as primers. As a result, a PCR product including Para and EFmvaE gene and a PCR product including EFmvaS and Ttrp were obtained. A plasmid pMW219 (manufactured by Nippon Gene Co., Ltd.) was digested with SmaI in accordance with a common method. After the SmaI digestion, the PCR product containing pMW219 and purified Para and EFmvaE gene and the PCR product containing EFmvaS gene and Ttrp were ligated using In-Fusion HD Cloning Kit (manufactured by Clontech Laboratories, Inc.). The obtained plasmid was named pMW-Para-mvaES-Ttrp.

### 1-2-1) Construction of integrative conditional replication plasmid having upstream and downstream genes of mevalonic acid pathway

In order to construct an integrative plasmid having upstream and downstream genes of the mevalonic acid pathway, pAH162- λattL-TcR-λattR vector (Minaeva NI et al., BMC Biotechnol. 2008; 8: 63) was used.

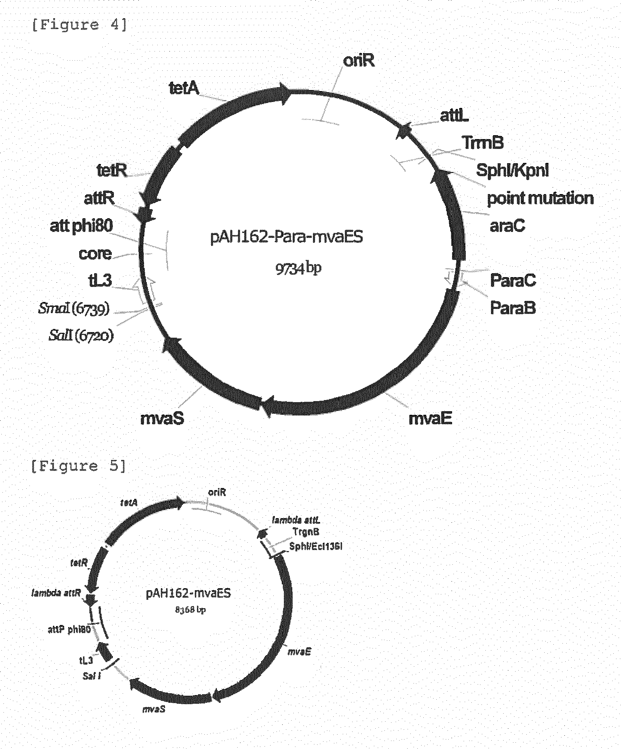

The KpnI-SalI fragment of pMW-Para-mvaES-Ttrp was cloned into the SphI-SalI recognition site of pAH162-λattL-TcR-λattR. As a result, a pAH162-Para-mvaES plasmid having the *E. faecalis*-derived mvaES operon under the control of the *E. coli* Para promoter and the repressor gene araC was constructed (FIG. 4).

In order to obtain a promoter deficient variant of the operon, the Ecll36II-SalI fragment of pMW219-Para-mvaES-Ttrp was subcloned into the same integrative vector. The map of the obtained plasmid is illustrated in FIG. 5.

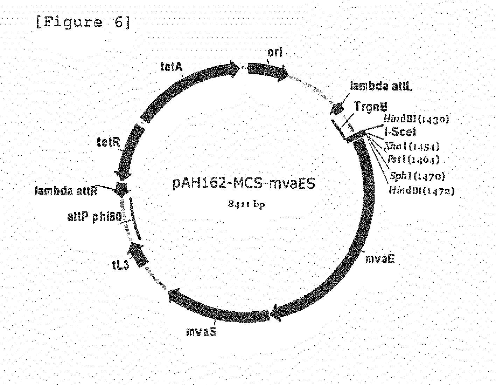

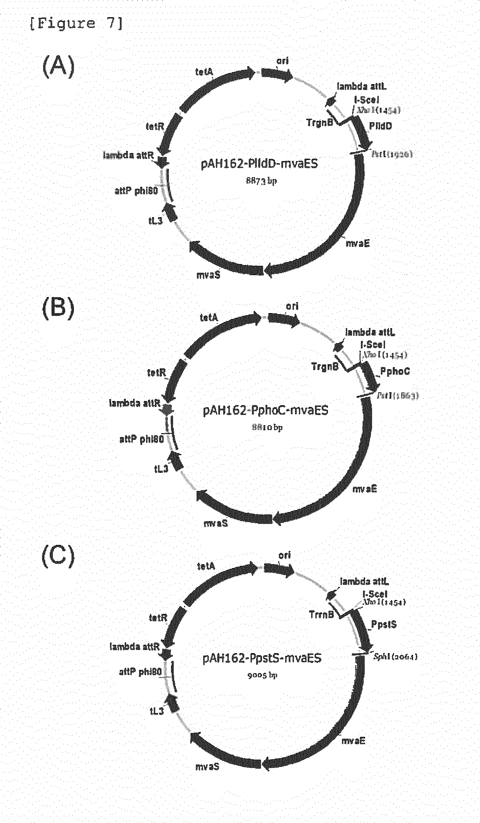

A set of plasmids for chromosome immobilization holding the mvaES gene under the control of different promoters was constructed. For this purpose, a polylinker including I-SceI, XhoI, PstI, and SphI recognition sites was inserted into a unique HindIII recognition site located upstream of the mvaES gene. In order to achieve this purpose, annealing was carried out using primers 1 and 2 (Table 5) and polynucleotide kinase. The obtained doublestranded DNA fragment was 5'-phosphorylated with polynucleotide kinase and the obtained phosphorylated fragment was inserted into the pAH162-mvaES plasmid cut with HindIII by ligation reaction. The resulting pAH162-MCS-mvaES plasmid (FIG. 6) was convenient for cloning the promoter while maintaining the desired orientation in front of the mvaES gene. DNA fragments holding the regulatory regions of lldD, phoC, and pstS genes were generated by PCR using the genomic DNA of *P. ananatis* SC17(0) strain (Katashkina JI et al., BMC Mol Biol., 2009; 10: 34) as a template and primers 3 and 4, primers 5 and 6, and primers 7 and 8 (Table 1), respectively, and cloned into an appropriate the restriction enzyme recognition site of pAH162-MCS-mvaES. The obtained plasmid is illustrated in FIG. 7. Sequence of the cloned promoter fragment was determined to confirm that the plasmid exactly corresponds to the expected nucleotide sequence.

### 1-2-2) Construction of plasmid for pAH162-Km-P_{tac}-KDyI chromosome immobilization

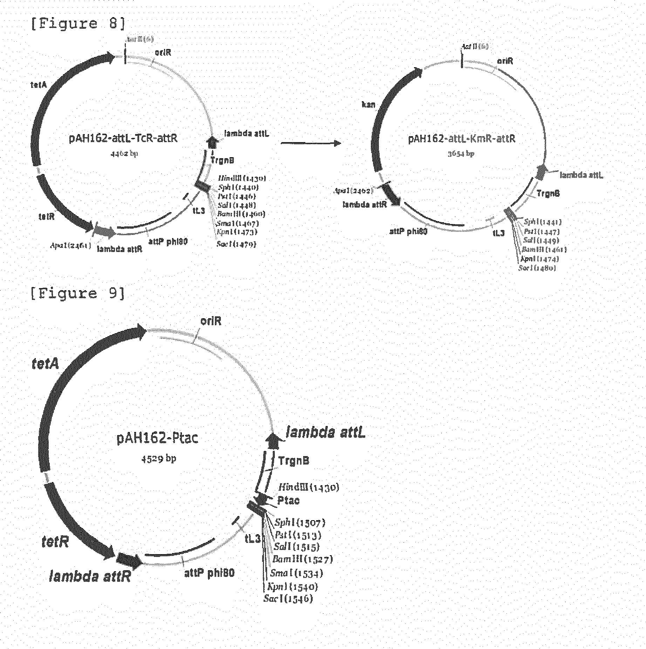

The AatII-ApaI fragment of pAH162-λattL-TcR-λattR containing the tetAR gene (Minaeva NI et al., BMC Biotechnol., 2008; 8: 63) was replaced by the DNA fragment obtained by PCR using primers 9 and 10 (Table 1) and a pUC4K plasmid (Taylor LA And Rose RE., Nucleic Acids Res., 16, 358, 1988) as templates. As a result, pAH162-λattL-Km^{R}-λattR was obtained (FIG. 8).

The P_{tac} promoter was inserted into the HindIII-SphI recognition site of the pAH162-λattL-Tc^{R}-λattR vector (Minaeva NI et al., BMC Biotechnol., 2008; 8: 63). As a result, an expression vector for chromosome immobilization pAH162-P_{tac} was constructed. The sequence of the cloned promoter fragment was determined and the cloned promoter fragment was confirmed that the sequence was as designed. The map of pAH162-P_{tac} is illustrated in FIG. 9.

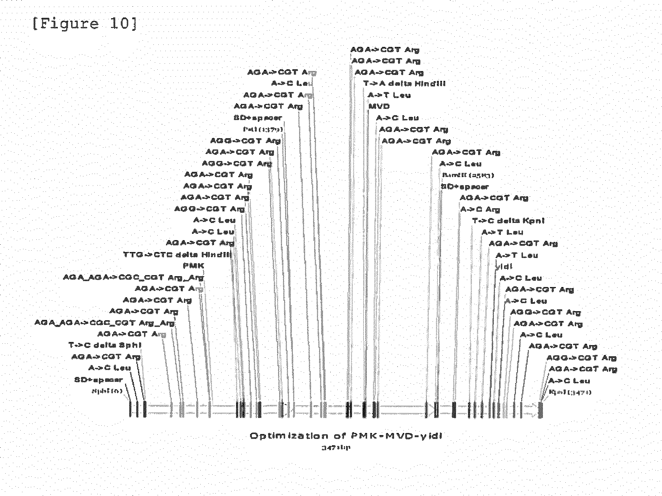

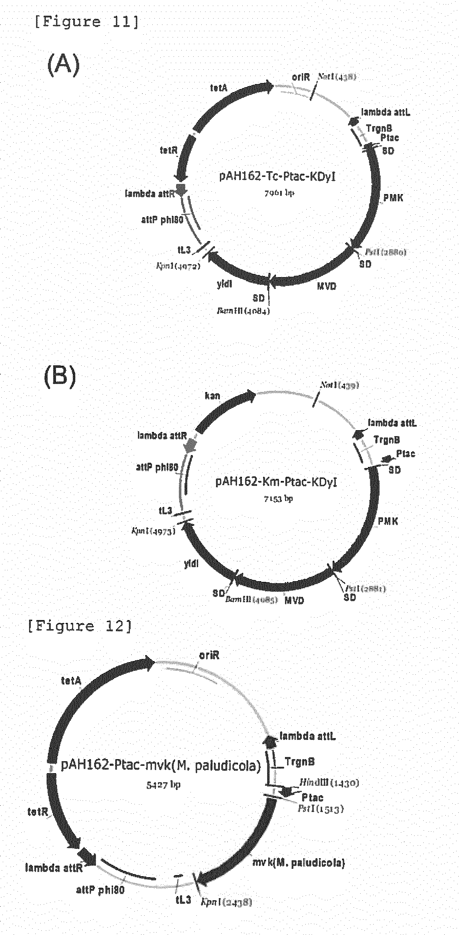

The DNA fragment (FIG. 10) holding PMK, MVD and yIDI genes originated from *S. cerevisiae* that was chemically synthesized by ATG Service Gene (Russia) and in which a rare codon was replaced by a synonymous codon was subcloned into the SphI-KpnI restriction enzyme recognition site of the vector for chromosome immobilization pAH162-Ptac. The DNA sequence including the chemically synthesized KDyI operon is shown in SEQ ID NO: 43. The obtained plasmid pAH162-Tc-Ptac-KDyI holding the P_{tac}-KDyI expression cassette is illustrated in FIG. 11(A). Thereafter, in order to replace the drug resistance marker gene, the NotI-KpnI fragment of pAH162-Tc-P_{tac}-KDyI holding the tetAR gene was replaced by the corresponding fragment with pAH162-λattL-KmR-λattR. As a result, a pAH162-Km-P_{tac}-KDyI plasmid having a kanamycin resistance gene kan as a marker was obtained (FIG. 11(B)).

A chemically synthesized DNA fragment including the coding part of the putative mvk gene originated from SANAE [for complete genomic sequence, see GenBank Accession No. AP011532] being *Methanocella paludicola* strain ligated to the classical SD sequence was cloned into the PstI-KpnI recognition site of the integrative expression vector pAH162-P_{tac}. The map of the chromosome-immobilized plasmid having the mvk gene is illustrated in FIG. 12.

### 1-3) Construction of recipient strain SC17(0) ΔampC::attB_{Phi80} ΔampH::attBₚₕᵢ₈₀ Δcrt::P_{tac}-mvk (M. paludicola)

ΔampH::attB_{Phi80} and ΔampC::attBₚₕᵢ₈₀ chromosomal alterations were introduced stepwise into a *P. ananatis* SC17(0) strain using a two stage procedures of λRed-dependent integration of a PCR amplified DNA fragment including the kan gene adjacent to attLₚₕᵢ₈₀ and attRₚₕᵢ₈₀ and a 40bp sequence homologous to the target chromosome site (Katashkina JI et al., BMC Mol Biol., 2009; 10: 34) and subsequent phage phi80 Int/Xis-dependent removal of the kanamycin resistance marker (Andreeva IG et al., FEMS Microbiol Lett., 2011; 318 (1): 55-60). SC17(0) is a λRed resistant derivative of *P. ananatis* AJ13355 (Katashkina JI et al., BMC Mol Biol., 2009; 10: 34). The annotated complete genomic sequence of *P. ananatis* AJ13355 was capable of being used as PRJDA162073 or GenBank Accession Nos. AP012032.1 and AP012033.1. DNA fragments each used for integration into the ampH and ampC gene regions were generated using a pMWattphi plasmid [Minaeva NI et al., BMC Biotechnol., 2008; 8: 63] as templates and using primers 11 and 12 and primers 13 and 14 (Table 1) as primers. Primers 15 and 16 and primers 17 and 18 (Table 1) were used for PCR verification of the obtained chromosome modification product.

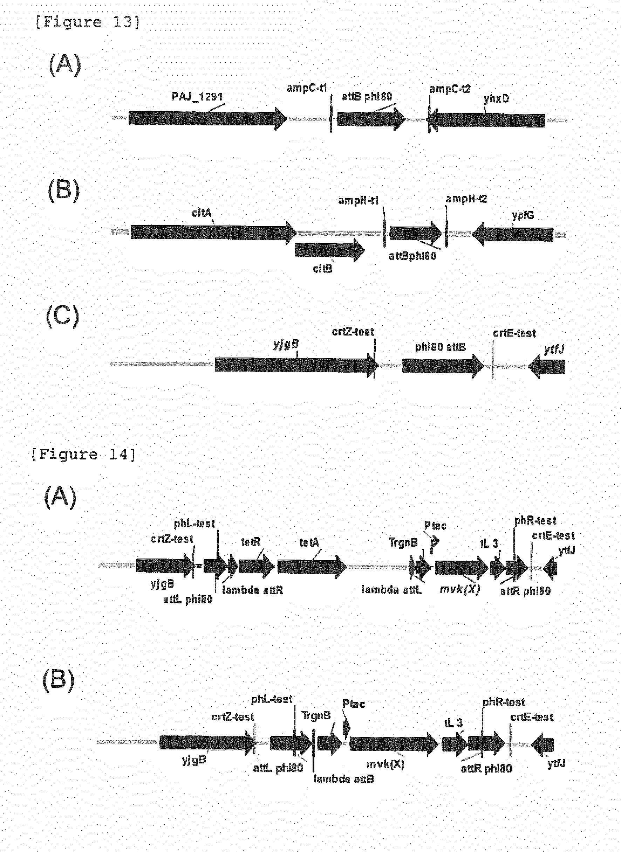

In parallel, a derivative of *P. ananatis* SC17(0) holding the attB site of phi80 phage instead of the crt operon located on pEA320 320 kb mega plasmid, which was a part of *P. ananatis* AJ13355 genome was constructed. In order to obtain this strain, λRed-dependent integration of the PCR amplified DNA fragments holding attLₚₕᵢ₈₀-kan-attRₚₕᵢ₈₀ adjacent to a 40bp region homologous to the target site in the genome was carried out using the previously described procedure (Katashkina JI et al., BMC Mol Biol., 2009; 10: 34). The DNA fragment used for replacement of the crt operon by attLₚₕᵢ₈₀-kan-attRₚₕᵢ₈₀ was amplified by reaction using primers 19 and 20 (Table 1). The pMWattphi plasmid (Minaeva NI et al., BMC Biotechnol., 2008; 8: 63) was used as a template in this reaction. The obtained integrant was named SC17(0)Δcrt::attLₚₕᵢ₈₀-kan-attR_{phi/80}. Primers 21 and 22 (Table 1) were used for the PCR verification of the chromosome structure of SC17 (0) Δcrt::attLₚₕᵢ₈₀-kan-attRₚₕᵢ₈₀. Removal of the kanamycin resistance marker from the constructed strain was performed using a pAH129-cat helper plasmid in accordance with the previously reported method (Andreeva IG et al., FEMS Microbiol Lett., 2011; 318 (1): 55-60). Oligonucleotides 21 and 22 were used for PCR validation of the obtained SC17(0)Δcrt::attBₚₕᵢ₈₀ strain. The obtained maps of ΔampC::attBₚₕᵢ₈₀, ΔampH::attBₚₕᵢ₈₀, and Δcrt::attBₚₕᵢ₈₀ genome modification products are illustrated in FIGS. 13 (A), (B) and (C), respectively.

The above pAH162-Ptac-mvk (*M. paludicola*) plasmid was integrated at the attBₚₕᵢ₈₀ site of SC17(0)Δcrt::attBₚₕᵢ₈₀ in accordance with the previously reported protocol (Andreeva IG et al., FEMS Microbiol Lett., 2011; 318 (1): 55-60). The plasmid integration was confirmed by polymerase chain reaction using primers 21 and 23, and primers 22 and 24 (Table 1). As a result, SC17(0)Δcrt::pAH162-P_{tac}-mvk (*M. paludicola*) strain was obtained. A map of the Δcrt::pAH162-P_{tac}-mvk (*M*. *paludicola*) modification product is illustrated in FIG. 14(A).

Subsequently, the genetic trait of SC17(0)Δcrt::pAH162-P_{tac}-mvk (*M. paludicola*) was changed to SC17(0) ΔampC::attBₚₕᵢ₈₀ ΔampH::attBₚₕᵢ₈₀ through a genomic DNA electroporation method (Katashkina JI et al., BMC Mol Biol., 2009; 10: 34). The obtained strain uses a tetracycline resistance gene tetRA as a marker. The vector part of the pAH162-Ptac-mvk (*M. paludicola*) integrative plasmid including the tetRA marker gene was removed using the previously reported pMW-intxis-cat helper plasmid [Katashkina JI et al., BMC Mol Biol., 2009; 10: 34]. As a result, a marker gene deficient strain SC17(0) ΔampH::attB_{φ80} ΔampC::attB_{φ80} Δcrt::P_{tac}-mvk (*M. paludicola*) was obtained. The map of Δcrt::P_{tac}-mvk (*M*. *paludicola*) genome modification product is illustrated in FIG. 14(B).

### 1-4) Construction of SWITCH strain set

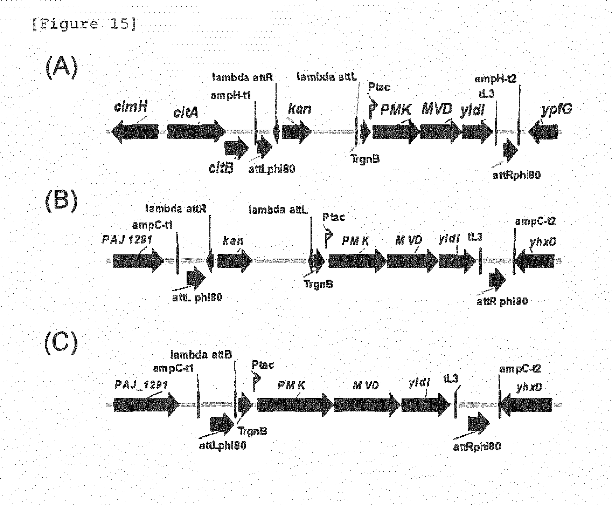

The pAH162-Km-Ptac-KDyI plasmid was integrate into the chromosome of SC17 (0) ΔampH::attB_{φ80} ΔampC::attB_{φ80} Acrt::P_{tac}-mvk (M. paludicola)/pAH123-cat strain in accordance with the previously reported protocol (Andreeva IG et al. FEMS Microbiol Lett. 2011; 318 (1): 55-60). The cells were seeded on LB agar containing 50 mg/L kanamycin. The grown Km^{R} clones were tested in PCR reactions using primers 11 and 15 and primers 11 and 17 (Table 1). The strains holding the pAH162-Km-Ptac-KDyI plasmid integrated into ΔampH::attB_{φ80} or AampC::attB_{φ80}m were selected. The maps of ΔampH::pAH162-Km-Ptac-KDyI and ΔampC::pAH162-Km-Ptac-KDyI chromosome modification products are illustrated in FIGS. 15(A) and 15(B).

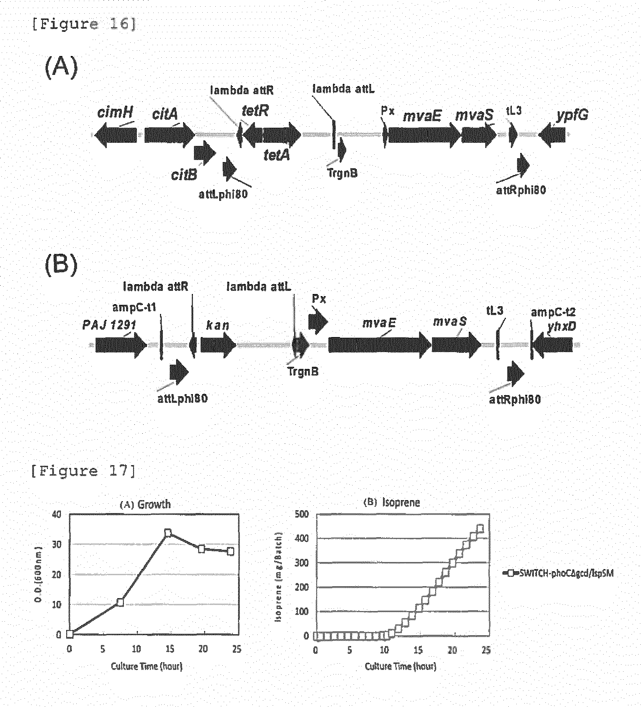

pAH162-Px-mvaES (here, Px is one of the following regulatory regions: araC-Pₐᵣₐ (*E. coli*), P_{11dD}, P_{phoC}, or P_{pstS}) was inserted into the attBphi80 sites of SC17(0) ΔampC::pAH162-Km-P_{tac}-KDyI ΔampH::attBₚₕᵢ₈₀ Δcrt::P_{tac}-mvk (*M*. *paludicola*) and SC17(0) ΔampC::attB_{Phi80} ΔampH::pAH162-Km-P_{tac}-KDyI Δcrt::P_{tac}-mvk (*M. paludicola*) recipient strains in accordance with the previously reported protocol [Andreeva IG et al., FEMS Microbiol Lett., 2011; 318 (1): 55-60] using a pAH123-cat helper plasmid. As a result, two sets of strains named SWITCH-Px-1 and SWITCH-Px-2 were obtained. The map of ΔampH::pAH162-Px-mvaES and ΔampC::pAH162-Px-mvaES chromosome modification products are illustrated in FIG. 16

### [Table 1]

**Table 1. Primer sequences used in Preparation Example 1**

| No. | Name | Sequence 5' -> 3' |
|---|---|---|
| 1 | Linker-F | AGCTTTAGGGATAACAGGGTAATCTCGAGCTGCAGGCATGCA (SEQ ID NO: 19) |
| 2 | Linker-R | AGCTTGCATGCCTGCAGCTCGAGATTACCCTGTTATCCCTAA (SEQ ID NO: 20) |
| 3 | 11dD5' CAS | TTTTAAGCTTTAGGGATAACAGGGTAATCTCGAGATTTAAAGCGGCTGCTTTAC (SEQ ID NO : 21) |
| 4 | 11dD3' CAS | TTTTTAAGCTTGCATGCCTGCAGTATTTAATAGAATCAGGTAG (SEQ ID NO: 22) |
| 5 | phoC5' CAS | TTTTTAAGCTTTAGGGATAACAGGGTAATCTCGAGTGGATAACCTCATGTAAAC (SEQ ID NO: 23) |
| 6 | phoC3' CAS | TTTTTAAGCTTGCATGCCTGCAGTTGATGTCTGATTATCTCTGA (SEQ ID NO: 24) |
| 7 | pstS5' CAS | TTTTTAAGCTTTAGGGATAACAGGGTAATCTCGAGAGCCTCTCACGCGTGAATC (SEQ ID NO: 25) |
| 8 | pstS3' CAS | TTTTTAAGCTTGCATGCCTGCAGAGGGGGAGAAAAGTCAGGCTAA (SEQ ID NO: 26) |
| 9 | n67 | TGCGAAGACGTCCTCGTGAAGAAGGTGTTGCTG (SEQ ID NO: 27) |
| 10 | n68 | TGCAAGGGCCCCGTTGTGTCTCAAAATCTCGATG (SEQ ID NO: 28) |
| 11 | ampH-attL-phi80 | ATGCGCACTCCTTACGTACTGGCTCTACTGGTTTCTTTGCGAAAGGTCATTTTTCCTGAATATGCTC ACA (SEQ ID NO: 29) |
| 12 | ampH-attR-phi80 | TTAAGGAATCGCCTGGACCATCATCGGCGAGCCGTTCTGACGTTTGTTGACAGCTGGTCCAATG ( SEQ ID NO: 30) |
| 13 | DampC-phL | CTGATGAACTGTCACCTGAATGAGTGCTGATGAAAATATAGAAAGGTCATTTTTCCTGAATATGCTC A (SEQ ID NO: 31) |
| 14 | DampC-phR | ATTCGCCAGCATAACGATGAAGCTGTTGAGCTGAGGAACACGTTTGTTGAGCTGGTCCAATG ( SEQ ID NO: 32) |
| 15 | ampH-t1 | GCGAAGCCCTCTCCGTTG (SEQ ID NO: 33) |
| 16 | ampH-t2 | AGCCAGTCAGCCTCATCAGCT (SEQ ID NO: 34) |
| 17 | ampC-t1 | GATTCCCACTTCACCGAGCCG (SEQ ID NO: 35) |
| 18 | ampC-t2 | GGCAGGTATGGTGCCTGACG (SEQ ID NO: 36) |
| 19 | crtE-attRphi80 | ATGACGGTCTGCGCAAAAAAACACGTTCATCTCACTCGCGCGTTTGTTGACAGCTGGTCCAATG ( SEQ ID NO: 37) |
| 20 | crtZ-attLphi80 | ATGTTGTGGATTTGGAATGCCCTGATCGTTTTCGTTACCGGAAAGGTCATTTTTCCTGAATATGCTC A(SEQ ID NO: 38) |
| 21 | crtZ-test | CCGTGTGGTTCTGAAAGCCGA (SEQ ID NO: 39) |
| 22 | crtE-test | CGTTGCCGTAAATGTATCCGT (SEQ ID NO: 40) |
| 23 | phL-test | GGATGTAAACCATAACACTCTGCGAAC (SEQ ID NO: 41) |
| 24 | phR-test | GATTGGTGGTTGAATTGTCCGTAAC (SEQ ID NO: 42) |

### 1-5) Introduction of isoprene synthase expression plasmid

In accordance with a common method, electrocompetent cells of SWITCH bacteria were prepared and pSTV28-Ptac-IspSM (WO2013/179722) being an expression plasmid of *Mucuna bracteata*-derived isoprene synthase was introduced by electroporation. The obtained isoprenoid compound-producing microorganisms each were named SWITCH-Para/IspSM, SWITCH-Plld/IspSM, SWITCH-PpstS/IspSM, and SWITCH-PphoC/IspSM.

### Preparation Example 2: Construction of SC17(0)Δgcd and SWITCH-PphoCΔgcd and introduction of isoprene synthase

It has been known that the gcd gene of *P. ananatis* encodes glucose dehydrogenase and *P. ananatis* accumulates gluconic acid during aerobic growth (Andreeva IG et al., FEMS Microbiol Lett. 2011 May; 318 (1): 55-60).

The SC17(0)Δgcd strain in which the gcd gene was disrupted was constructed by λRed-dependent integration of DNA fragments obtained by PCR using primers gcd-attL, and gcd-attR (Table 2), and the pMW118-attL-kan-attR plasmid as a template (Minaeva NI et al., BMC Biotechnol. 2008 8: 63). Primers gcd-t1 and gcd-t2 (Table 2) were used to confirm the integrants.

The genomic DNA of SC17(0)Δgcd strain was isolated using Wizard Genomic DNA Purification Kit (Promega Corporation), and the electric genetic transformation was carried out into a derivative not containing the marker of the SWITCH-PphoC strain in accordance with the method reported previously [Katashkina JI et al., BMC Mol Biol. 2009; 10: 34]. As a result, a SWITCH-PphoC-Δgcd (KmR) strain was obtained. Primers gcd-t1 and gcd-t2 (Table 2) were used for PCR analysis of the obtained integrants. The kanamycin resistance marker gene was obtained in accordance with a standard λIng/Xis mediated method [Katashkina JI et al., BMC Mol Biol. 2009; 10: 34]. The obtained strain was named SWITCH-PphoC Δgcd strain.

Competent cells of the SWITCH-PphoC Δgcd strain were prepared in accordance with the standard method and pSTV28-Ptac-IspSM (WO 2013/179722) being a vector for expression of isoprene synthase originated from *Mucuna bracteata* was introduced by electroporation. The obtained isoprenoid compound-producing microorganism was named SWITCH-PphoC Δgcd/IspSM.

### [Table 2]

**Table 2. List of primers**

| Primer | NUCLEOTIDE SEQUENCE (SEQ ID NO.) |
|---|---|
| gcd-attL | GGTCAACATTATGGGGAAAAACTCCTCATCCTTTAGCGTGTGAAGCTGCTTTTTTATACTAAGTTGG (SEQ ID NO: 1) |
| gcd-attR | TTACTTCTGGTCGGGCAGCGCATAGGCAATCACGTAATCGCGCTCAAGTTAGTATAAAAAAGCTGAAC (SEQ ID NO: 2) |
| gcd-t1 | TGACAACAATCTATCTGATT (SEQ ID NO: 3) |
| gcd-t2 | TGCGCCTGGTTAAGCTGGCG (SEQ ID NO: 4) |

### Example 1 Culture evaluation of SWITCH-PphoC Δgcd/IspSM

### 1) Jar culture conditions of the isoprene-producing microorganism SWITCH-PphoCAgcd/IspSM strain

Jar culture was carried out for bacterial growth of the isoprene-producing microorganism SWITCH-PphoCAgcd/IspSM strain. For the jar culture, a 3 L volume gas stirring fermentation device (manufactured by ABLE Corporation) was used. The glucose culture medium was adjusted so as to have the composition listed in Table 3. As preculture, the isoprene-producing microorganism SWITCH-PphoCAgcd/IspSM strain was applied onto an LB plate containing chloramphenicol (60 mg/L) and was cultured at 34°C for 16 hours. 2 L of glucose culture medium was charged in the 3 L volume gas stirring fermentation device and bacterial cells in seven well-grown plates were inoculated to start the culture. The gas stirring fermentation device includes the draft tube located inside and the gas supply pipe supplying gas to the inside of the culture tank. The gas supply pipe was provided with the gas discharge part made of the sintered metal film having an average pore diameter of 5.0 µm. The culture conditions were a pH of 6.8 (controlled with ammonia gas) and 34°C. 2.3 L/min of air (oxygen concentration: 20% (v/v)) was fed into the culture medium so that the discharge gas linear velocity at the gas discharge part made of the sintered metal film was 0.03 m/sec. The dissolved oxygen (DO) concentration in the culture liquid was measured using a galvanic type DO sensor (manufactured by ABLE Corporation) and the culture was carried out for 48 hours. During the cultivation, glucose adjusted to 500 g/L was continuously added so that the glucose concentration in the culture medium was 15 g/L or more.

### [Table 3]

**Table 3. Glucose culture medium composition**

| Group A | (Terminal concentration) |
|---|---|
| Glucose | 80 g/L |
| MgSO₄, 7aq | 2.0 g/L |

| Group B | |
|---|---|
| (NH₄)₂SO₄ | 2.0 g/L |
| KH₂PO₄ | 2.0 g/L |
| FeSO₄.7aq | 20 mg/L |
| MnSO₄.5aq | 20 mg/L |
| Yeast Extract | 4.0 g/L |

After 1 L of Group A and Group B were adjusted, heat sterilize was carried out at 120°C for 20 min. After Group A and Group B were allowed to stand to cool, Group A and Group B were mixed at 1:1 and chloramphenicol (60 mg/L) was added. The resultant mixture was used as a culture medium.

### 2) Method for measuring oxygen and isoprene concentration in fermentation discharge gas

The concentration of isoprene in the fermentation discharge gas was measured using a multi-gas analyzer (F10, manufactured by GASERA Ltd.). The concentrations of oxygen and carbonic acid gas in the fermentation discharge gas were measured using a gas analyzer (DEX-1562A, manufactured by ABLE Corporation).

### Example 2 Culture evaluation of SWITCH-PphoC Δgcd/IspSM

The culture test of the isoprene-producing microorganism SWITCH-PphoC Δgcd/IspSMstrain was carried out using the gas stirring fermentation device. For the culture, the 3 L volume gas stirring fermentation device (type BMA-02PI, manufactured by ABLE Corporation) was used. The gas stirring fermentation device included a culture tank, a draft tube located inside the culture tank, and a gas supply pipe supplying gas to the inside of the culture tank, in which the gas supply pipe was provided with a gas discharge part made of the sintered metal film having an average pore diameter of 5.0 µm. The inner diameter D₁ of the culture tank was 7.6 cm, the inner diameter D₂ of the draft tube was 5.5 cm, the height H₂ₘₐₓ of the uppermost part of the draft tube from the bottom of the culture tank was 33.1 cm, and the height H₂ₘᵢₙ of the lowermost part of the draft tube from the bottom of the culture tank was 9.8 cm.

The glycerol stock of SWITCH-PphoCAgcd/IspSM strain was thawed and 50 µL of the bacterial cell suspension was uniformly applied to six LB plates containing 60 mg/L of chloramphenicol, followed by culturing at 34°C for 16 hours as preculture.

Subsequently, 2.0 L of the fermentation medium was poured into the culture tank of the gas stirring fermentation device. Here, the fermentation medium was prepared by preparing 1 L of each Group A and Group B listed in Table 4, thereafter sterilizing Group A and Group B by heating at 120°C for 20 minutes, allowing Group A and Group B stand to cool, thereafter mixing Group A and Group B at 1:1, and adding chloramphenicol (60 mg / L). The whole amount of the bacterial cells corresponding to the obtained six preculture plates was inoculated into the fermentation medium and the culture was started. The liquid level height H_{L} of the culture liquid from the bottom of the culture tank was 34.8 cm.

The culture temperature was set to 34°C and sterilized air of 2.3 L/min (oxygen concentration: 21% (v/v)) was fed into the fermentation medium so that the discharge gas linear velocity at the gas discharge part made of the sintered metal film was 0.03 m/sec. The pH of the fermentation medium was controlled to 6.8 using ammonia gas and the culture was carried out for 24 hours while the DO concentration in the fermentation medium was being measured using a galvanic type DO sensor (SDOU-10L160-125, manufactured by ABLE Corporation). The DO concentration at the start of culture before inoculation was defined to be 21% and the DO concentration in the saturated sodium sulfite solution was defined to be 0%. The ammonia gas for pH adjustment was supplied from the upper part of the fermentation device by connecting a gas supply pipe exclusively used for supplying the ammonia gas to the fermentation medium and the culture temperature was controlled with a silicone rubber heater and cooling water. During the culture, a glucose culture medium adjusted to 700 g/L containing 0.07 mL/L of DISFOAM GD-113K was added continuously so that the glucose concentration in the fermentation medium was ranging from 20 g/L to 40 g/L.

### [Table 4]

**Table 4. Fermentation medium composition**

| Group A | |
|---|---|
| Glucose | 80 g/L |
| W₄. 7aq | 2. 0 g/L |
| Without adjusting pH | |
| | |

| Group B | |
|---|---|
| Trisodium Citrate | 2. 0 g/L |
| (NH₄)₂SO₄ | 2.0 g/L |
| KH₂PO₄ | 2.0 g/L |
| FeSO₄.7aq | 20 mg/L |
| MnSO₄.5aq | 20 mg/L |
| Yeast Extract | 4. 0 g/L |
| DISFOAM GD-113K | 0.02 mL/L |
| Without adjusting pH | |

After the culture was started, sampling was adequately carried out and the O. D. value and glucose concentration were analyzed. The concentration of isoprene in the fermentation discharge gas was measured using a multi-gas analyzer (F10, manufactured by GASERA Ltd.) and the concentrations of oxygen and carbonic acid gas in the fermentation discharge gas were measured using a gas analyzer (DEX-1562A, manufactured by ABLE Corporation). After the fermentation broth was diluted by 101-fold, the O. D. value was measured at 600 nm with a spectrophotometer (U-2900, manufactured by Hitachi High-Tech Sciences Corporation).

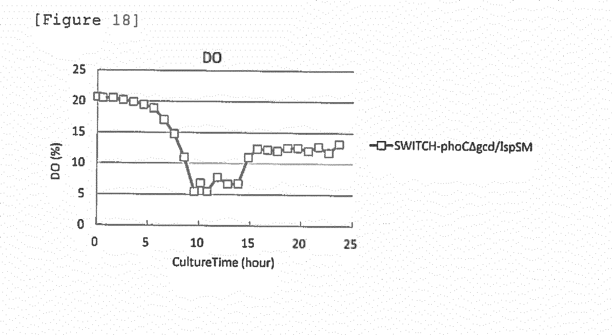

The O. D. value and isoprene production amount after the culture of the SWITCH-PphoC Δgcd strain/IspSM strain are listed in Table 5. The graphs of change over time of the O. D. value and isoprene production amount during the culture are illustrated in FIG. 17(A) and FIG. 17(B), respectively. The graph of change over time of the DO concentration in the fermentation medium is illustrated in FIG. 18.

### [Table 5]

**Table 5. O. D. Value and isoprene production amount after culture**

| Strain | O. D. (600nm) | Isoprene (mg/Batch) |
|---|---|---|
| SWITCH-PphoC Δgcd/IspSM | 27.7 | 440 |

In the 24-hour culture, the SWITCH-PphoC Δgcd/IspSM strain consumed 364 g of glucose and the production amount of isoprene was 440 mg. The DO concentration in the fermentation medium was maintained at 5% or more over the whole period of the culture. From these results, it was demonstrated that the chemical substance (isoprene) was capable of being produced by using a gas stirring fermentation device including the culture tank, the draft tube located inside the culture tank, and the gas supply pipe supplying the gas to the inside of the culture tank, in which the gas supply pipe was provided with the gas discharge part made of the sintered metal film. Reference Signs List

1 Culture Tank
2 Draft Tube
3 Gas Supply Pipe
4 Gas Discharge Part Made of Sintered Metal Film
5 Culture Liquid
10 Gas Stirring Fermentation Device

## Claims

1. A gas stirring fermentation device comprising:
a culture tank;
a draft tube located inside the culture tank; and
a gas supply pipe supplying gas to inside of the culture tank, wherein
the gas supply pipe is provided with a gas discharge part made of a sintered metal film, and
a culture liquid in the culture tank is capable of being stirred by gas discharged from the gas discharge part.

2. The device according to claim 1, wherein the sintered metal film has an average pore diameter of 20 µm or less.

3. The device according to claim 1 or 2, wherein a discharge gas linear velocity (Gas discharge amount [m³/s]/Surface area [m²] of sintered metal film) at the gas discharge part made of the sintered metal film is 0.04 m/s or less.

4. The device according to any one of claims 1 to 3, wherein D₁ and D₂ satisfy the relation of 0.7<D₂/D₁ where D₁ is the inner diameter of the culture tank and D₂ is the inner diameter of the draft tube.

5. A method for producing a chemical substance, the method comprising:
a step of discharging gas from a gas discharge part made of a sintered metal film into a culture liquid including microorganisms having ability to produce the chemical substance and passing the culture liquid including the discharged gas through inside of a draft tube to stir the culture liquid, and
a step of culturing the microorganisms to produce the chemical substance.

6. The method according to claim 5, wherein the chemical substance comprises a flammable substance.

7. The method according to claim 5 or 6, wherein the chemical substance is a hydrophobic substance.

8. The method according to any one of claims 5 to 7, wherein the chemical substance is an isoprenoid compound.

9. The method according to any one of claims 5 to 8, wherein the sintered metal film has an average pore diameter of 20 µm or less.

10. The method according to any one of claims 5 to 9, wherein a discharge gas linear velocity (Gas discharge amount [m³/s]/Surface area [m²] of sintered metal film) at the gas discharge part made of the sintered metal film is 0.04 m/s or less.


SEQUENCE LISTING

<110>  Ajinomoto Co., Inc.

<120>  Gas Mixing Fermentation Apparatus

<130>  PAMA-281361

<160>  43

<170>  PatentIn version 3.5

<210>  1
<211>  68
<212>  DNA
<213>  Artificial Sequence

<220>
<223>  Primer (gcd-attL)

<400>  1
ggtcaacatt atggggaaaa actcctcatc ctttagcgtg tgaagcctgc ttttttatac      60

taagttgg                                                                68

<210>  2
<211>  68
<212>  DNA
<213>  Artificial Sequence

<220>
<223>  Primer (gcd-attR)

<400>  2
ttacttctgg tcgggcagcg cataggcaat cacgtaatcg cgctcaagtt agtataaaaa      60

agctgaac                                                                68

<210>  3
<211>  20
<212>  DNA
<213>  Artificial Sequence

<220>
<223>  Primer (gcd-t1)

<400>  3
tgacaacaat ctatctgatt                                                   20

<210>  4
<211>  20
<212>  DNA
<213>  Artificial Sequence

<220>
<223>  Primer (gcd-t2)

<400>  4
tgcgcctggt taagctggcg                                                   20

<210>  5
<211>  803
<212>  PRT
<213>  Enterococcus faecalis

<400>  5

Met Lys Thr Val Val Ile Ile Asp Ala Leu Arg Thr Pro Ile Gly Lys
1               5                   10                  15

Tyr Lys Gly Ser Leu Ser Gln Val Ser Ala Val Asp Leu Gly Thr His
            20                  25                  30

Val Thr Thr Gln Leu Leu Lys Arg His Ser Thr Ile Ser Glu Glu Ile
        35                  40                  45

Asp Gln Val Ile Phe Gly Asn Val Leu Gln Ala Gly Asn Gly Gln Asn
    50                  55                  60

Pro Ala Arg Gln Ile Ala Ile Asn Ser Gly Leu Ser His Glu Ile Pro
65                  70                  75                  80

Ala Met Thr Val Asn Glu Val Cys Gly Ser Gly Met Lys Ala Val Ile
                85                  90                  95

Leu Ala Lys Gln Leu Ile Gln Leu Gly Glu Ala Glu Val Leu Ile Ala
            100                 105                 110

Gly Gly Ile Glu Asn Met Ser Gln Ala Pro Lys Leu Gln Arg Phe Asn
        115                 120                 125

Tyr Glu Thr Glu Ser Tyr Asp Ala Pro Phe Ser Ser Met Met Tyr Asp
    130                 135                 140

Gly Leu Thr Asp Ala Phe Ser Gly Gln Ala Met Gly Leu Thr Ala Glu
145                 150                 155                 160

Asn Val Ala Glu Lys Tyr His Val Thr Arg Glu Glu Gln Asp Gln Phe
                165                 170                 175

Ser Val His Ser Gln Leu Lys Ala Ala Gln Ala Gln Ala Glu Gly Ile
            180                 185                 190

Phe Ala Asp Glu Ile Ala Pro Leu Glu Val Ser Gly Thr Leu Val Glu
        195                 200                 205

Lys Asp Glu Gly Ile Arg Pro Asn Ser Ser Val Glu Lys Leu Gly Thr
    210                 215                 220

Leu Lys Thr Val Phe Lys Glu Asp Gly Thr Val Thr Ala Gly Asn Ala
225                 230                 235                 240

Ser Thr Ile Asn Asp Gly Ala Ser Ala Leu Ile Ile Ala Ser Gln Glu
                245                 250                 255

Tyr Ala Glu Ala His Gly Leu Pro Tyr Leu Ala Ile Ile Arg Asp Ser
            260                 265                 270

Val Glu Val Gly Ile Asp Pro Ala Tyr Met Gly Ile Ser Pro Ile Lys
        275                 280                 285

Ala Ile Gln Lys Leu Leu Ala Arg Asn Gln Leu Thr Thr Glu Glu Ile
    290                 295                 300

Asp Leu Tyr Glu Ile Asn Glu Ala Phe Ala Ala Thr Ser Ile Val Val
305                 310                 315                 320

Gln Arg Glu Leu Ala Leu Pro Glu Glu Lys Val Asn Ile Tyr Gly Gly
                325                 330                 335

Gly Ile Ser Leu Gly His Ala Ile Gly Ala Thr Gly Ala Arg Leu Leu
            340                 345                 350

Thr Ser Leu Ser Tyr Gln Leu Asn Gln Lys Glu Lys Lys Tyr Gly Val
        355                 360                 365

Ala Ser Leu Cys Ile Gly Gly Gly Leu Gly Leu Ala Met Leu Leu Glu
    370                 375                 380

Arg Pro Gln Gln Lys Lys Asn Ser Arg Phe Tyr Gln Met Ser Pro Glu
385                 390                 395                 400

Glu Arg Leu Ala Ser Leu Leu Asn Glu Gly Gln Ile Ser Ala Asp Thr
                405                 410                 415

Lys Lys Glu Phe Glu Asn Thr Ala Leu Ser Ser Gln Ile Ala Asn His
            420                 425                 430

Met Ile Glu Asn Gln Ile Ser Glu Thr Glu Val Pro Met Gly Val Gly
        435                 440                 445

Leu His Leu Thr Val Asp Glu Thr Asp Tyr Leu Val Pro Met Ala Thr
    450                 455                 460

Glu Glu Pro Ser Val Ile Ala Ala Leu Ser Asn Gly Ala Lys Ile Ala
465                 470                 475                 480

Gln Gly Phe Lys Thr Val Asn Gln Gln Arg Leu Met Arg Gly Gln Ile
                485                 490                 495

Val Phe Tyr Asp Val Ala Asp Ala Glu Ser Leu Ile Asp Glu Leu Gln
            500                 505                 510

Val Arg Glu Thr Glu Ile Phe Gln Gln Ala Glu Leu Ser Tyr Pro Ser
        515                 520                 525

Ile Val Lys Arg Gly Gly Gly Leu Arg Asp Leu Gln Tyr Arg Ala Phe
    530                 535                 540

Asp Glu Ser Phe Val Ser Val Asp Phe Leu Val Asp Val Lys Asp Ala
545                 550                 555                 560

Met Gly Ala Asn Ile Val Asn Ala Met Leu Glu Gly Val Ala Glu Leu
                565                 570                 575

Phe Arg Glu Trp Phe Ala Glu Gln Lys Ile Leu Phe Ser Ile Leu Ser
            580                 585                 590

Asn Tyr Ala Thr Glu Ser Val Val Thr Met Lys Thr Ala Ile Pro Val
        595                 600                 605

Ser Arg Leu Ser Lys Gly Ser Asn Gly Arg Glu Ile Ala Glu Lys Ile
    610                 615                 620

Val Leu Ala Ser Arg Tyr Ala Ser Leu Asp Pro Tyr Arg Ala Val Thr
625                 630                 635                 640

His Asn Lys Gly Ile Met Asn Gly Ile Glu Ala Val Val Leu Ala Thr
                645                 650                 655

Gly Asn Asp Thr Arg Ala Val Ser Ala Ser Cys His Ala Phe Ala Val
            660                 665                 670

Lys Glu Gly Arg Tyr Gln Gly Leu Thr Ser Trp Thr Leu Asp Gly Glu
        675                 680                 685

Gln Leu Ile Gly Glu Ile Ser Val Pro Leu Ala Leu Ala Thr Val Gly
    690                 695                 700

Gly Ala Thr Lys Val Leu Pro Lys Ser Gln Ala Ala Ala Asp Leu Leu
705                 710                 715                 720

Ala Val Thr Asp Ala Lys Glu Leu Ser Arg Val Val Ala Ala Val Gly

725                 730                 735

Leu Ala Gln Asn Leu Ala Ala Leu Arg Ala Leu Val Ser Glu Gly Ile
            740                 745                 750

Gln Lys Gly His Met Ala Leu Gln Ala Arg Ser Leu Ala Met Thr Val
        755                 760                 765

Gly Ala Thr Gly Lys Glu Val Glu Ala Val Ala Gln Gln Leu Lys Arg
    770                 775                 780

Gln Lys Thr Met Asn Gln Asp Arg Ala Leu Ala Ile Leu Asn Asp Leu
785                 790                 795                 800

Arg Lys Gln

<210>  6
<211>  2412
<212>  DNA
<213>  Enterococcus faecalis

<400>  6
atgaaaacag tagttattat tgatgcatta cgaacaccaa ttggaaaata taaaggcagc      60

ttaagtcaag taagtgccgt agacttagga acacatgtta caacacaact tttaaaaaga     120

cattccacta tttctgaaga aattgatcaa gtaatctttg gaaatgtttt acaagctgga     180

aatggccaaa atcccgcacg acaaatagca ataaacagcg gtttgtctca tgaaattccc     240

gcaatgacgg ttaatgaggt ctgcggatca ggaatgaagg ccgttatttt ggcgaaacaa     300

ttgattcaat taggagaagc ggaagtttta attgctggcg ggattgagaa tatgtcccaa     360

gcacctaaat tacaacgttt taattacgaa acagaaagct acgatgcgcc tttttctagt     420

atgatgtatg atggattaac ggatgccttt agtggtcagg caatgggctt aactgctgaa     480

aatgtggccg aaaagtatca tgtaactaga gaagagcaag atcaattttc tgtacattca     540

caattaaaag cagctcaagc acaagcagaa gggatattcg ctgacgaaat agccccatta     600

gaagtatcag gaacgcttgt ggagaaagat gaagggattc gccctaattc gagcgttgag     660

aagctaggaa cgcttaaaac agtttttaaa gaagacggta ctgtaacagc agggaatgca     720

tcaaccatta atgatggggc ttctgctttg attattgctt cacaagaata tgccgaagca     780

cacggtcttc cttatttagc tattattcga gacagtgtgg aagtcggtat tgatccagcc     840

tatatgggaa tttcgccgat taaagccatt caaaaactgt tagcgcgcaa tcaacttact     900

acggaagaaa ttgatctgta tgaaatcaac gaagcatttg cagcaacttc aatcgtggtc     960

caaagagaac tggctttacc agaggaaaag gtcaacattt atggtggcgg tatttcatta    1020

ggtcatgcga ttggtgccac aggtgctcgt ttattaacga gtttaagtta tcaattaaat 1080

caaaaagaaa agaaatatgg agtggcttct ttatgtatcg gcggtggctt aggactcgct 1140

atgctactag agagacctca gcaaaaaaaa aacagccgat tttatcaaat gagtcctgag 1200

gaacgcctgg cttctcttct taatgaaggc cagatttctg ctgatacaaa aaaagaattt 1260

gaaaatacgg ctttatcttc gcagattgcc aatcatatga ttgaaaatca aatcagtgaa 1320

acagaagtgc cgatgggcgt tggcttacat ttaacagtgg acgaaactga ttatttggta 1380

ccaatggcga cagaagagcc ctcagttatt gcggctttga gtaatggtgc aaaaatagca 1440

caaggattta aaacagtgaa tcaacaacgc ttaatgcgtg gacaaatcgt tttttacgat 1500

gttgcagatc ccgagtcatt gattgataaa ctacaagtaa gagaagcgga agtttttcaa 1560

caagcagagt taagttatcc atctatcgtt aaacggggcg gcggcttaag agatttgcaa 1620

tatcgtactt ttgatgaatc atttgtatct gtcgactttt tagtagatgt taaggatgca 1680

atgggggcaa atatcgttaa cgctatgttg gaaggtgtgg ccgagttgtt ccgtgaatgg 1740

tttgcggagc aaaagatttt attcagtatt ttaagtaatt atgccacgga gtcggttgtt 1800

acgatgaaaa cggctattcc agtttcacgt ttaagtaagg ggagcaatgg ccgggaaatt 1860

gctgaaaaaa ttgttttagc ttcacgctat gcttcattag atccttatcg ggcagtcacg 1920

cataacaaag gaatcatgaa tggcattgaa gctgtagttt tagctacagg aaatgataca 1980

cgcgctgtta gcgcttcttg tcatgctttt gcggtgaagg aaggtcgcta ccaaggcttg 2040

actagttgga cgctggatgg cgaacaacta attggtgaaa tttcagttcc gcttgcttta 2100

gccacggttg gcggtgccac aaaagtctta cctaaatctc aagcagctgc tgatttgtta 2160

gcagtgacgg atgcaaaaga actaagtcga gtagtagcgg ctgttggttt ggcacaaaat 2220

ttagcggcgt tacgggcctt agtctctgaa ggaattcaaa aaggacacat ggctctacaa 2280

gcacgttctt tagcgatgac ggtcggagct actggtaaag aagttgaggc agtcgctcaa 2340

caattaaaac gtcaaaaaac gatgaaccaa gaccgagcca tggctatttt aaatgattta 2400

agaaaacaat aa 2412

<210> 7
<211> 2412
<212> DNA
<213> Artificial Sequence

<220>
<223> DNA having modified codons, which encodes mvaE derived from
Enterococcus faecalis

<400> 7
atgaaaaccg tggttattat cgatgcgctg cgcacgccga ttggtaaata taaaggcagc 60

ctgtctcaag tgagcgccgt tgatctgggt acgcatgtga ccacgcagct gctgaaacgt 120

cacagcacca tctctgaaga aattgatcag gtgatctttg gtaacgttct gcaagccggt 180

aatggtcaga atccggcacg tcagattgca atcaacagtg gcctgagcca tgaaattccg 240

gcgatgaccg tgaatgaagt ttgcggtagc ggcatgaaag cggttattct ggccaaacag 300

ctgatccagc tgggtgaagc ggaagtgctg attgccggcg gtatcgaaaa catgagtcag 360

gcaccgaaac tgcaacgttt taattatgaa accgaaagct acgatgcccc gttcagctct 420

atgatgtatg atggcctgac cgatgcattt agcggtcagg cgatgggcct gacggcagaa 480

aacgtggcgg aaaaatacca tgttacccgc gaagaacagg atcagttttc tgttcacagt 540

cagctgaaag cggcccaggc ccaggcagaa ggtattttcg ccgatgaaat cgcaccgctg 600

gaagtgtctg gtacgctggt tgaaaaagat gaaggcattc gtccgaatag tagcgtggaa 660

aaactgggca ccctgaaaac ggtgttcaaa gaagatggca ccgttacggc gggcaatgca 720

agcaccatca atgatggtgc gagtgccctg attatcgcga gccaggaata tgcagaagcg 780

catggcctgc cgtacctggc cattatccgc gattctgtgg aagttggtat tgatccggca 840

tatatgggca ttagtccgat caaagcgatt cagaaactgc tggcccgtaa ccagctgacc 900

accgaagaaa ttgatctgta cgaaatcaat gaagcgtttg cagcgaccag tattgtggtt 960

cagcgcgaac tggccctgcc ggaagaaaaa gttaacattt atggcggtgg catcagcctg 1020

ggtcacgcaa ttggtgccac cggtgcacgt ctgctgacca gtctgagcta tcagctgaat 1080

cagaaagaga aaaaatacgg tgtggcaagc ctgtgtattg gtggcggtct gggtctggcc 1140

atgctgctgg aacgtccgca gcagaagaaa aactctcgtt tttaccagat gagtccggaa 1200

gaacgtctgg ccagtctgct gaacgaaggc cagattagcg cagataccaa aaaagaattc 1260

gaaaatacgg cactgtctag tcagatcgcg aaccatatga ttgaaaatca gatcagcgaa 1320

accgaagtgc cgatgggtgt tggcctgcac ctgaccgtgg atgaaacgga ttatctggtt 1380

ccgatggcga cggaagaacc gagcgttatt gccgcactgt ctaacggtgc aaaaatcgcg 1440

cagggcttta aaaccgtgaa tcagcagcgt ctgatgcgcg gccagattgt gttctacgat 1500

gttgcggatc cggaaagcct gatcgataaa ctgcaagtgc gcgaagccga agtttttcag 1560

caggcagaac tgagctatcc gtctattgtg aaacgtggcg gtggcctgcg cgatctgcaa 1620

taccgtacct ttgatgaaag tttcgtgagc gttgatttcc tggtggatgt taaagatgcc 1680

atgggtgcaa acatcgtgaa tgcgatgctg gaaggcgttg ccgaactgtt tcgtgaatgg 1740

ttcgcggaac agaaaatcct gttttctatc ctgagtaact acgcgaccga aagcgtggtt 1800

accatgaaaa cggccattcc tgtgagccgc ctgtctaaag gtagtaatgg ccgtgaaatt 1860

gcggaaaaaa tcgttctggc gagccgctat gcctctctgg atccgtaccg tgccgtgacc 1920

cataacaaag gtattatgaa tggcatcgaa gcagtggttc tggcgaccgg taacgatacc 1980

cgtgccgtgt ctgcaagttg ccatgcattc gcagttaaag aaggtcgtta tcagggcctg 2040

accagctgga cgctggatgg tgaacagctg atcggcgaaa tttctgtgcc gctggccctg 2100

gcaaccgtgg gtggcgcgac gaaagttctg ccgaaaagcc aggcggccgc agatctgctg 2160

gcggtgaccg atgcaaaaga actgtctcgc gtggttgcgg ccgttggtct ggcacagaat 2220

ctggcagcgc tgcgtgcgct ggtgtctgaa ggtattcaga aaggccacat ggcactgcaa 2280

gcccgtagtc tggccatgac cgtgggtgca acgggcaaag aagtggaagc agttgcgcag 2340

cagctgaaac gccagaaaac catgaaccag gatcgtgcca tggcaatcct gaatgatctg 2400

cgcaaacagt aa 2412

<210> 8
<211> 383
<212> PRT
<213> Enterococcus faecalis

<400> 8

Met Thr Ile Gly Ile Asp Lys Ile Ser Phe Phe Val Pro Pro Tyr Tyr
1               5                   10                  15

Ile Asp Met Thr Ala Leu Ala Glu Ala Arg Asn Val Asp Pro Gly Lys
            20                  25                  30

Phe His Ile Gly Ile Gly Gln Asp Gln Met Ala Val Asn Pro Ile Ser
        35                  40                  45

Gln Asp Ile Val Thr Phe Ala Ala Asn Ala Ala Glu Ala Ile Leu Thr
    50                  55                  60

Lys Glu Asp Lys Glu Ala Ile Asp Met Val Ile Val Gly Thr Glu Ser
65                  70                  75                  80

Ser Ile Asp Glu Ser Lys Ala Ala Ala Val Val Leu His Arg Leu Met
                85                  90                  95

Gly Ile Gln Pro Phe Ala Arg Ser Phe Glu Ile Lys Glu Ala Cys Tyr
            100                 105                 110

Gly Ala Thr Ala Gly Leu Gln Leu Ala Lys Asn His Val Ala Leu His
        115                 120                 125

Pro Asp Lys Lys Val Leu Val Val Ala Ala Asp Ile Ala Lys Tyr Gly
    130                 135                 140

Leu Asn Ser Gly Gly Glu Pro Thr Gln Gly Ala Gly Ala Val Ala Met
145                 150                 155                 160

Leu Val Ala Ser Glu Pro Arg Ile Leu Ala Leu Lys Glu Asp Asn Val
                165                 170                 175

Met Leu Thr Gln Asp Ile Tyr Asp Phe Trp Arg Pro Thr Gly His Pro
            180                 185                 190

Tyr Pro Met Val Asp Gly Pro Leu Ser Asn Glu Thr Tyr Ile Gln Ser
        195                 200                 205

Phe Ala Gln Val Trp Asp Glu His Lys Lys Arg Thr Gly Leu Asp Phe
    210                 215                 220

Ala Asp Tyr Asp Ala Leu Ala Phe His Ile Pro Tyr Thr Lys Met Gly
225                 230                 235                 240

Lys Lys Ala Leu Leu Ala Lys Ile Ser Asp Gln Thr Glu Ala Glu Gln
                245                 250                 255

Glu Arg Ile Leu Ala Arg Tyr Glu Glu Ser Ile Ile Tyr Ser Arg Arg
            260                 265                 270

Val Gly Asn Leu Tyr Thr Gly Ser Leu Tyr Leu Gly Leu Ile Ser Leu
        275                 280                 285

Leu Glu Asn Ala Thr Thr Leu Thr Ala Gly Asn Gln Ile Gly Leu Phe
    290                 295                 300

Ser Tyr Gly Ser Gly Ala Val Ala Glu Phe Phe Thr Gly Glu Leu Val
305                 310                 315                 320

Ala Gly Tyr Gln Asn His Leu Gln Lys Glu Thr His Leu Ala Leu Leu
                325                 330                 335

Asp Asn Arg Thr Glu Leu Ser Ile Ala Glu Tyr Glu Ala Met Phe Ala
            340                 345                 350

Glu Thr Leu Asp Thr Asp Ile Asp Gln Thr Leu Glu Asp Glu Leu Lys
        355                 360                 365

Tyr Ser Ile Ser Ala Ile Asn Asn Thr Val Arg Ser Tyr Arg Asn
    370                 375                 380

<210>  9
<211>  1152
<212>  DNA
<213>  Enterococcus faecalis

<400>  9
atgacaattg ggattgataa aattagtttt tttgtgcccc cttattatat tgatatgacg        60

gcactggctg aagccagaaa tgtagaccct ggaaaatttc atattggtat tgggcaagac 120

caaatggcgg tgaacccaat cagccaagat attgtgacat ttgcagccaa tgccgcagaa 180

gcgatcttga ccaaagaaga taaagaggcc attgatatgg tgattgtcgg gactgagtcc 240

agtatcgatg agtcaaaagc ggccgcagtt gtcttacatc gtttaatggg gattcaacct 300

ttcgctcgct ctttcgaaat caaggaagct tgttacggag caacagcagg cttacagtta 360

gctaagaatc acgtagcctt acatccagat aaaaaagtct tggtcgtagc ggcagatatt 420

gcaaaatatg gcttaaattc tggcggtgag cctacacaag gagctggggc ggttgcaatg 480

ttagttgcta gtgaaccgcg cattttggct ttaaaagagg ataatgtgat gctgacgcaa 540

gatatctatg acttttggcg tccaacaggc cacccgtatc ctatggtcga tggtcctttg 600

tcaaacgaaa cctacatcca atcttttgcc caagtctggg atgaacataa aaaacgaacc 660

ggtcttgatt ttgcagatta tgatgcttta gcgttccata ttccttacac aaaaatgggc 720

aaaaaagcct tattagcaaa aatctccgac caaactgaag cagaacagga acgaatttta 780

gcccgttatg aagaaagtat cgtctatagt cgtcgcgtag gaaacttgta tacgggttca 840

ctttatctgg gactcatttc ccttttagaa aatgcaacga ctttaaccgc aggcaatcaa 900

attggtttat tcagttatgg ttctggtgct gtcgctgaat ttttcactgg tgaattagta 960

gctggttatc aaaatcattt acaaaaagaa actcatttag cactgctgga taatcggaca 1020

gaactttcta tcgctgaata tgaagccatg tttgcagaaa ctttagacac agacattgat 1080

caaacgttag aagatgaatt aaaatatagt atttctgcta ttaataatac cgttcgttct 1140

tatcgaaact aa 1152

<210> 10
<211> 1152
<212> DNA
<213> Artificial Sequence

<220>
<223> DNA having modified codons, which encodes mvaS derived from
      Enterococcus faecalis

<400> 10
atgaccattg gtatcgataa aattagcttt ttcgtgccgc cgtattacat cgatatgacg 60

gcgctggccg aagcacgtaa cgttgatccg ggcaaatttc atattggcat cggtcaggat 120

cagatggcgg tgaacccgat ttctcaggat atcgttacct tcgcggccaa tgcagcggaa 180

gcaattctga cgaaagaaga taaagaagcg attgatatgg tgatcgttgg caccgaaagc 240

tctatcgatg aaagtaaagc cgcagcggtg gttctgcacc gtctgatggg cattcagccg 300

tttgcgcgca gcttcgaaat caaagaagcc tgctatggcg cgaccgccgg tctgcaactg 360

gccaaaaacc atgtggcact gcacccggat aaaaaagttc tggtggttgc cgcagatatt 420

gcgaaatacg gtctgaatag cggcggtgaa ccgacccagg gtgcaggtgc cgtggcaatg     480

ctggttgcat ctgaaccgcg tattctggcg ctgaaagaag ataacgtgat gctgacccag     540

gatatctatg atttttggcg tccgaccggt catccgtacc cgatggtgga tggcccgctg     600

agtaatgaaa cctatattca gagcttcgcc caggtttggg atgaacataa aaaacgtacg     660

ggtctggatt ttgcggatta tgatgcactg gcgttccaca ttccgtacac caaaatgggc     720

aaaaaagcgc tgctggccaa aatcagcgat cagacggaag ccgaacagga acgtattctg     780

gcacgctatg aagaaagcat cgtgtactct cgtcgcgttg gcaacctgta taccggttct     840

ctgtacctgg gcctgattag tctgctggaa aacgcgacca cgctgacggc cggcaatcag     900

atcggtctgt tttcttatgg cagtggtgcc gtggcagaat ttttcaccgg tgaactggtt     960

gccggctacc agaaccatct gcaaaaagaa acccacctgg ccctgctgga taatcgcacg    1020

gaactgtcta ttgcagaata tgaagcaatg tttgcggaaa ccctggatac ggatatcgat    1080

cagaccctgg aagatgaact gaaatatagt attagcgcga tcaacaatac ggtgcgtagt    1140

taccgcaatt aa                                                        1152

<210>  11
<211>  40
<212>  DNA
<213>  Artificial Sequence

<220>
<223>  Primer for amplifying a fragment comprising Para composed of araC
       and ara BAD promoters from E.coli

<400>  11
tgaattcgag ctcggtaccc actcttcctt tttcaatatt                          40

<210>  12
<211>  40
<212>  DNA
<213>  Artificial Sequence

<220>
<223>  Primer for amplifying a fragment comprising Para composed of araC
       and ara BAD promoters from E.coli

<400>  12
ataataacca cggttttcat tttttataac ctccttagag                          40

<210>  13
<211>  40
<212>  DNA
<213>  Artificial Sequence

<220>
<223>  Primer for amplifying a fragment comprising EFmvaE gene

<400>  13

ctctaaggag gttataaaaa atgaaaaccg tggttattat                              40


<210>  14
<211>  55
<212>  DNA
<213>  Artificial Sequence

<220>
<223>  Primer for amplifying a fragment comprising EFmvaE gene

<400>  14
ttatcgatac caatggtcat gtttttttac ctcctttact gtttgcgcag atcat             55


<210>  15
<211>  55
<212>  DNA
<213>  Artificial Sequence

<220>
<223>  Primer for amplifying a fragment comprising EFmvaS gene

<400>  15
atgatctgcg caaacagtaa aggaggtaaa aaaacatgac cattggtatc gataa             55


<210>  16
<211>  40
<212>  DNA
<213>  Artificial Sequence

<220>
<223>  Primer for amplifying a fragment comprising EFmvaS gene

<400>  16
cagcggaact ggcggctccc ttaattgcgg taactacgca                              40


<210>  17
<211>  40
<212>  DNA
<213>  Artificial Sequence

<220>
<223>  Primer for amplifying a fragment comprising Ttrp

<400>  17
tgcgtagtta ccgcaattaa gggagccgcc agttccgctg                              40


<210>  18
<211>  38
<212>  DNA
<213>  Artificial Sequence

<220>
<223>  Primer for amplifying a fragment comprising Ttrp

<400>  18
gtcgactcta gaggatccct aatgagaatt agtcaaat                                38

<210>  19
<211>  42
<212>  DNA
<213>  Artificial Sequence

<220>
<223>  Primer (Linker-F)

<400>  19
agctttaggg ataacagggt aatctcgagc tgcaggcatg ca                          42


<210>  20
<211>  42
<212>  DNA
<213>  Artificial Sequence

<220>
<223>  Primer (Linker-R)

<400>  20
agcttgcatg cctgcagctc gagattaccc tgttatccct aa                          42


<210>  21
<211>  55
<212>  DNA
<213>  Artificial Sequence

<220>
<223>  Primer (lldD5' CAS)

<400>  21
tttttaagct ttagggataa cagggtaatc tcgagattta aagcggctgc tttac            55


<210>  22
<211>  43
<212>  DNA
<213>  Artificial Sequence

<220>
<223>  Primer (lldD3' CAS)

<400>  22
tttttaagct tgcatgcctg cagtatttaa tagaatcagg tag                         43


<210>  23
<211>  54
<212>  DNA
<213>  Artificial Sequence

<220>
<223>  Primer (phoC5' CAS)

<400>  23
tttttaagct ttagggataa cagggtaatc tcgagtggat aacctcatgt aaac             54


<210>  24
<211>  44
<212>  DNA

<213>  Artificial Sequence

<220>
<223>  Primer (phoC3′ CAS)

<400>  24
tttttaagct tgcatgcctg cagttgatgt ctgattatct ctga                        44


<210>  25
<211>  54
<212>  DNA
<213>  Artificial Sequence

<220>
<223>   Primer (pstS5′ CAS)

<400>  25
tttttaagct ttagggataa cagggtaatc tcgagagcct ctcacgcgtg aatc             54


<210>  26
<211>  44
<212>  DNA
<213>  Artificial Sequence

<220>
<223>  Primer (pstS3′ CAS)

<400>  26
tttttaagct tgcatgcctg cagaggggag aaaagtcagg ctaa                        44


<210>  27
<211>  33
<212>  DNA
<213>  Artificial Sequence

<220>
<223>  Primer (n67)

<400>  27
tgcgaagacg tcctcgtgaa gaaggtgttg ctg                                    33


<210>  28
<211>  36
<212>  DNA
<213>  Artificial Sequence

<220>
<223>  Primer (n68)

<400>  28
tgcgaagggc cccgttgtgt ctcaaaatct ctgatg                                 36


<210>  29
<211>  70
<212>  DNA
<213>  Artificial Sequence

<220>

<223>  Primer (ampH-attL-phi80)

<400>  29
atgcgcactc cttacgtact ggctctactg gtttctttgc gaaaggtcat ttttcctgaa      60

tatgctcaca                                                              70


<210>  30
<211>  64
<212>  DNA
<213>  Artificial Sequence

<220>
<223>  Primer (ampH-attR-phi80)

<400>  30
ttaaggaatc gcctggacca tcatcggcga gccgttctga cgtttgttga cagctggtcc      60

aatg                                                                    64


<210>  31
<211>  68
<212>  DNA
<213>  Artificial Sequence

<220>
<223>  Primer (DampC-phL)

<400>  31
ctgatgaact gtcacctgaa tgagtgctga tgaaaatata gaaaggtcat ttttcctgaa      60

tatgctca                                                                68


<210>  32
<211>  64
<212>  DNA
<213>  Artificial Sequence

<220>
<223>  Primer (DampC-phR)

<400>  32
attcgccagc ataacgatgc cgctgttgag ctgaggaaca cgtttgttga cagctggtcc      60

aatg                                                                    64


<210>  33
<211>  18
<212>  DNA
<213>  Artificial Sequence

<220>
<223>   Primer (ampH-t1)

<400>  33
gcgaagccct ctccgttg                                                     18


<210>  34

<211>  21
<212>  DNA
<213>  Artificial Sequence

<220>
<223>  Primer (ampH-t2)

<400>  34
agccagtcag cctcatcagc g                                                21


<210>  35
<211>  21
<212>  DNA
<213>  Artificial Sequence

<220>
<223>  Primer (ampC-t1)

<400>  35
gattcccact tcaccgagcc g                                                21


<210>  36
<211>  21
<212>  DNA
<213>  Artificial Sequence

<220>
<223>  Primer (ampC-t2)

<400>  36
ggcaggtatg gtgctctgac g                                                21


<210>  37
<211>  64
<212>  DNA
<213>  Artificial Sequence

<220>
<223>  Primer (crtE-attRphi80)

<400>  37
atgacggtct gcgcaaaaaa acacgttcat ctcactcgcg cgtttgttga cagctggtcc      60

aatg                                                                   64


<210>  38
<211>  68
<212>  DNA
<213>  Artificial Sequence

<220>
<223>  Primer (crtZ-attLphi80)

<400>  38
atgttgtgga tttggaatgc cctgatcgtt ttcgttaccg gaaaggtcat ttttcctgaa      60

tatgctca                                                               68

<210>  39
<211>  21
<212>  DNA
<213>  Artificial Sequence

<220>
<223>  Primer (crtZ-test)

<400>  39
ccgtgtggtt ctgaaagccg a                                                21


<210>  40
<211>  21
<212>  DNA
<213>  Artificial Sequence

<220>
<223>  Primer (crtE-test)

<400>  40
cgttgccgta aatgtatccg t                                                21


<210>  41
<211>  27
<212>  DNA
<213>  Artificial Sequence

<220>
<223>  Primer (phL-test)

<400>  41
ggatgtaaac cataacactc tgcgaac                                          27


<210>  42
<211>  25
<212>  DNA
<213>  Artificial Sequence

<220>
<223>  Primer (phR-test)

<400>  42
gattggtggt tgaattgtcc gtaac                                            25


<210>  43
<211>  3471
<212>  DNA
<213>  Artificial Sequence

<220>
<223>  Sequence of the chemically synthesized DNA fragment retaining
       artificial KDyI operon with optimized codons

<400>  43
gcatgcagga ggtatgaatg tcagagttgc gtgccttcag tgccccaggg aaagcgttac      60

tcgctggtgg atatttagtt ttagatacaa aatatgaagc atttgtagtc ggattatcgg     120

cacgtatgca cgctgtagcc catccttacg gttcattgca agggtctgat aagtttgaag     180

tgcgtgtgaa aagtaaacaa tttaaagatg gggagtggct gtaccatata agtcctaaaa 240

gtggcttcat tcctgtttcg ataggcggat ctaagaaccc tttcattgaa aaagttatcg 300

ctaacgtatt tagctacttt aaacctaaca tggacgacta ctgcaatcgt aacttgttcg 360

ttattgatat tttctctgat gatgcctacc attctcagga ggatagcgtt accgaacatc 420

gtggcaaccg ccgtttgagt tttcattcgc accgtattga agaagttccc aaaacagggc 480

tgggctcctc ggcaggttta gtcacagttt taactacagc tttggcctcc ttttttgtat 540

cggacctgga aaataatgta gacaaatatc gtgaagttat tcataattta gcacaagttg 600

ctcattgtca agctcagggt aaaattggaa gcgggtttga tgtagcggcg gcagcatatg 660

gatctatccg ttatcgccgt ttcccacccg cattaatctc taatttgcca gatattggaa 720

gtgctactta cggcagtaaa ctggcgcatt tggttgatga agaagactgg aatattacga 780

ttaaaagtaa ccatttacct tcgggattaa ctttatggat gggcgatatt aagaatggtt 840

cagaaacagt aaaactggtc cagaaggtaa aaaattggta tgattcgcat atgccagaaa 900

gcctcaaaat atatacagaa ctcgatcatg caaattctcg ttttatggat ggactctcta 960

aactcgatcg cttacacgag actcatgacg attacagcga tcagatattt gagtctcttg 1020

agcgtaatga ctgtacctgt caaaagtatc ctgaaatcac agaagttcgt gatgcagttg 1080

ccacaattcg tcgttccttt cgtaaaataa ctaaagaatc tggtgccgat atcgaacctc 1140

ccgtacaaac tagcttattg gatgattgcc agaccttaaa aggagttctt acttgcttaa 1200

tacctggtgc tggtggttat gacgccattg cagtgattac taagcaagat gttgatcttc 1260

gtgctcaaac cgctaatgac aaacgttttt ctaaggttca atggctggat gtaactcagg 1320

ctgactgggg tgttcgtaaa gaaaaagatc cggaaactta tcttgataaa taactgcaga 1380

ggaggtatga atgaccgttt acacagcatc cgttaccgca cccgtcaaca tcgcaaccct 1440

taagtattgg gggaaacgtg acacgaagtt gaatctgccc accaattcgt ccatatcagt 1500

gactttatcg caagatgacc tccgtacgtt gacctctgcg gctactgcac ctgagtttga 1560

acgcgacact ttgtggttaa atggagaacc acacagcatc gacaatgaac gtactcaaaa 1620

ttgtctgcgc gacctccgcc aattacgtaa ggaaatggaa tcgaaggacg cctcattgcc 1680

cacattatct caatggaaac tccacattgt ctccgaaaat aactttccta cagcagctgg 1740

tttagcttcc tccgctgctg gctttgctgc attggtctct gcaattgcta agttatacca 1800

attaccacag tcaacttcag aaatatctcg tatagcacgt aaggggtctg gttcagcttg 1860

tcgttcgttg tttggcggat acgtggcctg ggaaatggga aaagctgaag atggtcatga 1920

ttccatggca gtacaaatcg cagacagctc tgactggcct cagatgaaag catgtgtcct 1980

tgttgtcagc gatattaaaa aggatgtgag ttccactcag ggtatgcaat tgaccgtggc 2040

aacctccgaa ctctttaaag aacgtattga acatgtcgta ccaaagcgtt ttgaagtcat 2100

gcgtaaagcc attgttgaaa aagatttcgc cacctttgca aaggaaacaa tgatggattc 2160

caactctttc catgccacat gtttggactc tttccctcca atattctaca tgaatgacac 2220

ttccaagcgt atcatcagtt ggtgccacac cattaatcag ttttacggag aaacaatcgt 2280

tgcatacacg tttgatgcag gtccaaatgc tgtgttgtac tacttagctg aaaatgagtc 2340

gaaactcttt gcatttatct ataaattgtt tggctctgtt cctggatggg acaagaaatt 2400

tactactgag cagcttgagg ctttcaacca tcaatttgaa tcatctaact ttactgcacg 2460

tgaattggat cttgagttgc aaaaggatgt tgcccgtgtg attttaactc aagtcggttc 2520

aggcccacaa gaaacaaacg aatctttgat tgacgcaaag actggtctcc caaaggaata 2580

aggatccagg aggtatgaat gactgccgac aacaatagta tgccccatgg tgcagtatct 2640

agttacgcca aattagtgca aaaccaaaca cctgaagaca ttttggaaga gtttcctgaa 2700

attattccat tacaacaacg tcctaatacc cgctctagtg agacgtcaaa tgacgaaagc 2760

ggagaaacat gtttttctgg tcatgatgag gagcaaatta agttaatgaa tgaaaattgt 2820

attgttttgg attgggacga taatgctatt ggtgccggca ccaagaaagt ttgtcattta 2880

atggaaaata ttgaaaaggg tttacttcat cgtgcattct ccgtctttat tttcaatgaa 2940

caaggtgaat tacttttaca acaacgtgcc actgaaaaaa taactttccc tgatctttgg 3000

actaacacat gctgctctca tccactttgt attgatgacg aattaggttt gaagggtaag 3060

ctcgacgata agattaaggg cgctattact gcggcggtgc gtaaactcga tcatgaatta 3120

ggtattccag aagatgaaac taagacacgt ggtaagtttc actttttaaa ccgtatccat 3180

tacatggcac caagcaatga accatggggt gaacatgaaa ttgattacat cctcttttat 3240

aagatcaacg ctaaagaaaa cttgactgtc aacccaaacg tcaatgaagt tcgtgacttc 3300

aaatgggttt caccaaatga tttgaaaact atgtttgctg acccaagtta caagtttacg 3360

ccttggttta agattatttg cgagaattac ttattcaact ggtgggagca attagatgac 3420

ctttctgaag tggaaaatga ccgtcaaatt catcgtatgc tctaaggtac c 3471